# EUROPEAN PATENT SPECIFICATION

(11) **EP 3 441 766 B1**
(45) Date of publication and mention of the grant of the patent: **11.03.2020**
(21) Application number: 17185959.8
(22) Date of filing: 11.08.2017
(51) Int. Cl.: G01N 33/533, G01N 33/542, A61K 47/68

(54) **NOVEL ANTIBODY CONJUGATES SUITABLE FOR USE IN DISCRETE FLUORESCENCE QUENCHING DISPLACEMENT IMMUNOASSAYS**
NEUARTIGE ANTIKÖRPERKONJUGATE ZUR VERWENDUNG IN DISKRETEN FLUORESZENZLÖSCHENDEN VERDRÄNGUNGSIMMUNOASSAYS
NOUVEAUX CONJUGUÉS D'ANTICORPS ADAPTÉS POUR ÊTRE UTILISÉS DANS DES DOSAGES IMMUNOLOGIQUES DE DÉPLACEMENT D'EXTINCTION DE FLUORESCENCE DISCRETS

(43) Date of publication of application: 13.02.2019
(73) Proprietor: Life Science Inkubator Sachsen GmbH & Co. KG, 01307 Dresden (DE)
(72) Inventor: BUETTNER, Karin, 01324 Dresden (DE); STUECKEMANN, Tom, 01309 Dresden (DE)
(74) Representative: Maikowski & Ninnemann Patentanwälte Partnerschaft mbB

(56) References cited:
- WO-A1-2014/065661
- WO-A1-2015/157446
- US-A1- 2015 361 090
- LE HOA THI ET AL: "Antibody functionalization with a dual reactive hydrazide/click crosslinker", ANALYTICAL BIOCHEMISTRY, ELSEVIER, AMSTERDAM, NL, vol. 435, no. 1, 8 January 2013 (2013-01-08), pages 68-73, XP028979481, ISSN: 0003-2697, DOI: 10.1016/J.AB.2012.12.018
- FISCHER-DURAND NATHALIE ET AL: "A new bioorthogonal cross-linker with alkyne and hydrazide end groups for chemoselective ligation. Application to antibody labelling", TETRAHEDRON, vol. 68, no. 47, 18 September 2012 (2012-09-18), - 18 September 2012 (2012-09-18), pages 9638-9644, XP028945120, ISSN: 0040-4020, DOI: 10.1016/J.TET.2012.09.062
- IGOR DOVGAN ET AL: "Acyl Fluorides: Fast, Efficient, and Versatile Lysine-Based Protein Conjugation via Plug-and-Play Strategy", BIOCONJUGATE CHEMISTRY, vol. 28, no. 5, 26 April 2017 (2017-04-26) , pages 1452-1457, XP055434855, US ISSN: 1043-1802, DOI: 10.1021/acs.bioconjchem.7b00141
- RESCHKE MELANIE L ET AL: "Multifunctionalization of cetuximab with bioorthogonal chemistries and parallel EGFR profiling of cell-lines using imaging, FACS and immunoprecipitation approaches", BIOCHIMICA ET BIOPHYSICA ACTA (BBA) - PROTEINS & PROTEOMICS, ELSEVIER, NETHERLANDS, vol. 1844, no. 12, 1 August 2014 (2014-08-01), pages 2182-2192, XP029091194, ISSN: 1570-9639, DOI: 10.1016/J.BBAPAP.2014.07.017
- GUHUAN LIU ET AL: "Doubly Caged Linker for AND-Type Fluorogenic Construction of Protein/Antibody Bioconjugates and In Situ Quantification", ANGEWANDTE CHEMIE INTERNATIONAL EDITION, vol. 56, no. 30, 17 July 2017 (2017-07-17) , pages 8686-8691, XP055467152, ISSN: 1433-7851, DOI: 10.1002/anie.201702748
- ADUMEAU PIERRE ET AL: "Site-Specifically Labeled Immunoconjugates for Molecular Imaging-Part 1: Cysteine Residues and Glycans", MOLECULAR IMAGING & BIOLOGY, ELSEVIER, BOSTON, vol. 18, no. 1, 11 January 2016 (2016-01-11), pages 1-17, XP035936455, ISSN: 1536-1632, DOI: 10.1007/S11307-015-0919-4 [retrieved on 2016-01-11]

## Description

### Field of the Invention

The present invention generally relates to the field of Fluorescence Quenching Immunoassays. Specifically, the invention provides novel antibody conjugates suitable for use in Discrete Fluorescence Quenching Displacement Immunoassays and methods for producing these antibody conjugates. The invention further relates to the use of the novel antibody conjugates and a kit comprising the same.

### Background Art

Antibodies are widely used molecular tools for clinical diagnostics, therapy and research (Chames, Van Regenmortel, Weiss, & Baty, 2009; Ecker, Jones, & Levine, 2015; Morgan & Levinsky, 1985; Scolnik, 2009; Siddiqui, 2010; Waldmann, 2003). In the field of immunodiagnostics, antibodies are used to quantify clinical biomarkers in complex biological samples like blood (Wild, 2013). These so called immunoassays can be discriminated between homogeneous and heterogeneous techniques (Wild, 2013). The heterogeneous immunoassays are today's gold standard for clinical diagnostics. Heterogeneous immunoassays use an antibody pair to capture and to detect the analyte (Wild, 2013). These assays reach good sensitivities, but require washing steps, which makes them complicated and slow.

In homogeneous immunoassays, the antibody reacts with the analyte in solution, which allows much faster assay times. Additionally, homogeneous immunoassays only require one analyte specific antibody, which makes them suitable for detecting smaller biomarkers. Because of their speed and simplicity homogeneous immunoassays are highly relevant for clinical diagnostics (Hemmil & Mukkala, 2001; Leuvering, Thai, Waart, & Schuurs, 1980; Nargessi, Landon, & Smith, 1979a; Ullman, Schwarzberg, & Rubenstein, 1976; Wild, 2013). However in contrast to heterogeneous immunoassays, homogeneous immunoassays have problems in signal generation, which so far results in less sensitive assays.

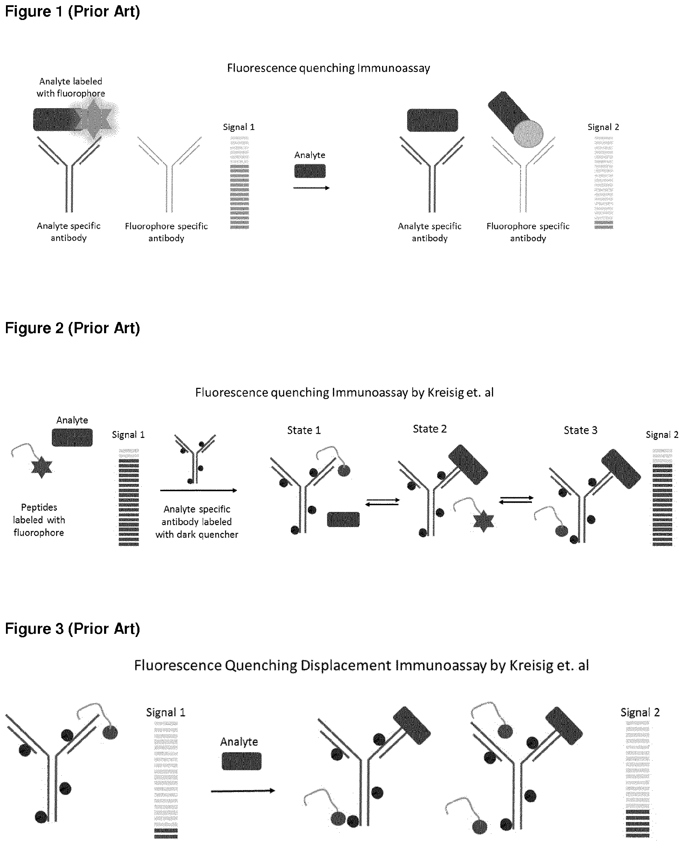

In most homogeneous immunoassays, the signal generating step is based on competition between the analyte and a known amount of fluorescently labeled analyte. The so-called *Fluorescence Quenching Immunoassay* uses two antibodies: one analyte specific antibody and one fluorophore specific antibody, whereby binding of the fluorophore specific antibody to the fluorophore reduces the fluorescence intensity. Both antibodies compete in binding the fluorescently labeled analyte. At steady state a given fluorescent signal is generated. By adding additional unlabeled analyte, via applying a sample, the steady state shifts and more fluorescence signal can be quenched by the fluorophore specific antibody. Figure 1 shows a scheme of a typical Fluorescence Quenching Immunoassay. The analyte concentration is, however, disproportionally to the fluorescence signal (Nargessi, Landon, & Smith, 1979b; Zuk, Rowley, & Ullman, 1979).

A modified version of the Fluorescence Quenching Immunoassay has been proposed by Kreisig et. al (Kreisig, Hoffmann, & Zuchner, 2011). This FRET-based homogeneous immunoassay consists of a dark-quencher labeled antibody and a fluorescently labeled peptide. Both the analyte and the peptide are mixed together and compete in binding the subsequently added antibody. When the peptide is bound by the antibody, the dark quencher and the fluorophore are in spatial proximity, resulting into a reduced fluorescence signal. In contrast, when the analyte is bound, the peptide is displaced and the attached fluorophore is no longer quenched and emits light. At steady state of this reaction, the emitted light is proportional to the analyte concentration, as shown in Figure 2. Because the steady state of this reaction can be reached already after 90 seconds, this assay is highly attractive for clinical point of care testing (POCT). However the FRET-based homogeneous Immunoassay from Kreisig et. al does not allow the quantification of biomarkers in the clinical relevant concentrations.

Additional publications from Kreisig et. al further describe the principle of a *Fluorescence Quenching Displacement Immunoassay.* In contrast to the *Fluorescence Quenching Immunoassay* in the *Fluorescence Quenching Displacement Immunoassay,* the dark quencher labeled antibody is premixed with the fluorophore labeled peptide. This pre-incubation step results into a lower starting signal, aiming to generate higher signal to noise ratios, which should increase the assay sensitivity. In order to generate a high analyte specific signal the added analyte consequently has to actively displace the bound peptide. However, Kreisig et. al could not show the functionality of the Fluorescence Quenching Displacement Immunoassay and it is therefore hypothesized that the signal generating step is problematic by using this method (Kreisig et al., 2013, 2015).

After analyzing the assay components, we further hypothesized that the dark quencher labeled antibody impedes the signal-generating step. Kreisig et. al generated the dark quencher labeled antibody by NHS-ester reactions. As a consequence of this bio-conjugation approach, dark quenchers are randomly distributed at the antibody surface, potentially resulting in undirected quenching of free diffusing peptides at steady state of the reaction (Fig. 3). Such undirected quenching of free diffusing peptides could explain both, the limited sensitivity of the *Fluorescence Quenching Immunoassay,* as well as the lack of functionality of the *Fluorescence Quenching Displacement Immunoassay.*

Several bio-conjugation techniques have been postulated, but no approach has been described to specifically generate an intramolecular interaction with the antigen binding site, without interfering with the antibodies affinity (Agarwal & Bertozzi, 2015; Kim, Ko, Park, & Lee, 2016; Kumar et al., 2015; Liberatore et al., 1990; Packard, Edidin, & Komoriya, 1986; Schumacher et al., 2015; Schumacher, Hackenberger, Leonhardt, & Helma, 2016; Sochaj, Swiderska, & Otlewski, 2015; Zimmerman et al., 2014).

Le, H. T. et al. (2013) discloses functionalized antibodies with a dual reactive ethynyl hydrazide/click crosslinker. The length of the crosslinker disclosed in D1 is fixed due to its defined chemical structure. The crosslinker disclosed in Le et al. is not capable of intramolecular binding to a molecule captured at the antigen binding site of the same antibody.

Fischer-Durand, N. et al. (2012) discloses an IgG-Coumarin dye conjugate, but does not tech the site of the antibody to which the cross-linker with alkyne and hydrazide end groups is conjugated.

Dovgan, I. et al. (2017) and RESCHKE M. L. et al. (2014) disclose antibody conjugates, in which a molecule of interest is coupled to a lysine residue of the antibody via click chemistry.

Liu, G. et al. (2017) discloses antibody conjugates, in which a molecule of interest is coupled to a cysteine residue of the antibody via click chemistry.

WO 2015/157446 A1 discloses site-specific antibody-drug-glycoconjugates, which are functionalized with at least one sialic acid moiety through enzymatic glycan remodelling. Sialylation of the antibody is achieved by contacting the antibody or fragment thereof with a functionalized CMP-sialic acid and a sialyltransferase and under conditions and for a time sufficient to attach at least one functionalized sialic acid to an N-linked oligosaccharide of the antibody or fragment thereof, such as an Fc fragment. An exemplary sialyltransferase is ST6Gal.

WO 2014/065661 A1 discloses antibody-conjugates, which comprise a core-GlcNAc-S(A)x substituent of formula (4): 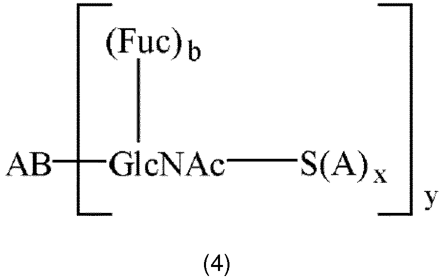 wherein AB in formula (4) represents an antibody. In order to produce such modified antibodies, monoclonal antibodies are first trimmed, i.e. deglycosylated with endo S from Streptomyces pyogenes and then subjected to an enzymatic introduction of a galactose derivative.

US 2015/361090 A1 discloses fluorophorelabeled, bi- or multi-functional, chemically reactive and/or biologically active conjugates, and related compositions and methods thereof.

### Description of the Invention

Accordingly, the problem of the invention is to provide a bio-conjugation technique, which overcomes the problems of the prior art, in particular which overcomes the limited sensitivity and the lack of functionality of known *Fluorescence Quenching Immunoassays.*

This problem is solved by the provision of a conjugate according to claim 1.

In a preferred embodiment, the invention provides a conjugate comprising an antibody bonded to a linker, characterized in that the length of the linker is adaptable such that the free terminus of the linker is capable of interacting with a molecule captured at the antibody binding site.

The term "antibody" is used in the broadest sense and specifically covers intact monoclonal antibodies, polyclonal antibodies, multispecific antibodies (e.g. bispecific antibodies) formed from at least two intact antibodies, and antibody fragments so long as they exhibit the desired biological activity. The antibody may be an IgM, IgG (e.g. IgG1, IgG2, IgG3 or IgG4), IgD, IgA or IgE, for example.

"Antibody fragments" comprise a portion of an intact antibody, generally the antigen binding or variable region of the intact antibody. Examples of antibody fragments include Fab, Fab', F(ab')2, and Fv fragments: diabodies; single-chain antibody molecules; and multispecific antibodies formed from antibody fragments.

The term "monoclonal antibody" as used herein refers to an antibody obtained from a population of substantially homogeneous antibodies, i.e. the individual antibodies comprising the population are identical except for possible naturally occurring mutations that may be present in minor amounts. Monoclonal antibodies are highly specific, being directed against a single antigenic site. Furthermore, in contrast to "polyclonal antibody" preparations which typically include different antibodies directed against different determinants (epitopes), each monoclonal antibody is directed against a single determinant on the antigen. In addition to their specificity, the monoclonal antibodies can frequently be advantageous in that they are synthesized by the hybridoma culture, uncontaminated by other immunoglobulins. The "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Köhler et al., Nature, 256:495 (1975), or may be made by generally well known recombinant DNA methods. The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein specifically include chimeric antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity.

"Humanized" forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain a minimal sequence derived from a non-human immunoglobulin. For the most part, humanized antibodies are human immunoglobulins (recipient antibody) in which residues from a complementarity-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, humanized antibodies may comprise residues which are found neither in the recipient antibody nor in the imported CDR or framework sequences.

These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, see Jones et al., Nature, 321:522-525 (1986), Reichmann et al, Nature. 332:323-329 (1988): and Presta, Curr. Op. Struct. Biel., 2:593-596 (1992). The humanized antibody includes a Primatized™ antibody wherein the antigen-binding region of the antibody is derived from an antibody produced by immunizing macaque monkeys with the antigen of interest.

"Single-chain Fv" or "sFv" antibody fragments comprise the VH and VL domains of antibody, wherein these domains are present in a single polypeptide chain. Generally, the Fv polypeptide further comprises a polypeptide linker between the VH and VL domains which enables the sFv to form the desired structure for antigen binding. For a review of sFv see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (VH) connected to a light-chain variable domain (VD) in the same polypeptide chain (VH - VD). By using a linker that is too short to allow pairing between the two domains on the same chain, the domains are forced to pair with the complementary domains of another chain and create two antigen-binding sites. Diabodies are described more fully in Hollinger et al., Proc. Natl. Acad. Sol. USA, 90:6444-6448 (1993). An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or non-proteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

As used herein, the expressions "cell", "cell line," and "cell culture" are used interchangeably and all such designations include progeny. Thus, the words "transformants" and "transformed cells" include the primary subject cell and culture derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, this will be clear from the context.

The terms "polypeptide", "peptide", and "protein", as used herein, are interchangeable and are defined to mean a biomolecule composed of amino acids linked by a peptide bond.

The terms "a", "an" and "the" as used herein are defined to mean "one or more" and include the plural unless the context is inappropriate.

In a preferred embodiment, any antibody that is already known in the art, may be used in the conjugate and/or methods of the invention.

In a more embodiment, the conjugate according to invention comprises a monoclonal or polyclonal antibody, preferably a monoclonal antibody. In a further preferred embodiment, the monoclonal antibody is a chimeric antibody.

In yet a further preferred embodiment, the antibody is an isolated antibody.

The "target polypeptide" can be any polypeptide, peptide, substrate, antigen or analyte, which may be the subject of investigation in a *Fluorescence Quenching Immunoassay.* The invention is not limited to a specific target polypeptide. Accordingly, the invention is also not limited to a specifically defined antibody. Antibodies have to be produced, e.g. with any technique known in the art, for the desired purpose to be comprised in a conjugate for use in a *Fluorescence Quenching Immunoassay* as described above. Alternatively, any antibody, preferably monoclonal antibody known in the art may be used.

An antibody, which may be comprised in the conjugate of the invention, may for example be selected from:
- suitable antibodies capable for detecting cardiac Troponin-I, (clinical marker to validate heart disorders), for example mouse monoclonal hybridoma clones selected from 8E10, C5, 4C2, 19C7, 16A11, 3C7, P4-14G5, M18, M155, MF4, 16A12, P4-9F6, 228, 625, 458, 267, 596, 84, 415, 581, 10F4, 247, 560, 17F3, p45-10, 916, 909, 820, 810, 801. These mouse monoclonal hybridoma clones are described in and can be ordered from HyTest Ltd., Catalog ID 4T21.
- suitable antibodies capable for detecting cardiac Troponin-T, (clinical marker to validate heart disorders), for example mouse monoclonal hybridoma clones 9G6, 7F4, 7G7, 2F3, 1A11, 1C11, 1F11, 7E7. These mouse monoclonal hybridoma clones are described in and can be ordered from HyTest Ltd., Catalog ID 4T19.
- suitable antibodies capable for detecting NT-ProBNP, (clinical marker to validate heart disorders), for example mouse monoclonal hybridoma clones. 5F11cc, 13G12cc, 15C4cc, 24E11cc, 29D12cc, 18H5cc. These mouse monoclonal hybridoma clones are described in and can be ordered from HyTest Ltd., Catalog ID 4NT1cc.
- suitable antibodies capable for detecting cardiac myoglobin, (clinical marker to validate heart disorders), for example mouse monoclonal hybridoma clones 4E2, 7C3, IB4. These mouse monoclonal hybridoma clones are described in and can be ordered from HyTest Ltd., Catalog ID 4M23.
- suitable antibodies capable for detecting D-dimer, (blood coagulation marker), for example mouse monoclonal hybridoma clones DD1, DD2, DD3, DD4, DD5, DD6, DD22, DD41, DD44, DD46, DD93, DD189, DD255. These mouse monoclonal hybridoma clones are described in and can be ordered from HyTest Ltd., Catalog ID 4D30.
- suitable antibodies capable for detecting Insulin, (clinical marker to validate metabolic syndrome), for example mouse monoclonal hybridoma clones C7C9, D4B8, 7F8, 3A6, 7F5. These mouse monoclonal hybridoma clones are described in and can be ordered from HyTest Ltd., Catalog ID 211.
- suitable antibodies capable for detecting C-Peptide, (clinical marker to validate metabolic syndrome), for example mouse monoclonal hybridoma clones 1H8, 2B7, 4H8, 5B8, 7E10, 2A11. These mouse monoclonal hybridoma clones are described in and can be ordered from HyTest Ltd., Catalog ID 2I2.
- suitable antibodies capable for detecting Cystatin-C, (clinical marker to validate kidney diseases), for example mouse monoclonal hybridoma clones Cyst10, Cyst13, Cyst16, Cyst18, Cyst19, Cyst23, Cyst24, Cyst28, Cyst11, Cyst20, Cyst29. These mouse monoclonal hybridoma clones are described in and can be ordered from HyTest Ltd., Catalog ID 4CC1.
- suitable antibodies capable for detecting C-reactive protein, (clinical marker to validate status of inflammation), for example mouse monoclonal hybridoma clones C1, C2, C3, C4, C5, C6, C7, CRP11, CRP30, CRP36, CRP103, CRP135, CRP169. These mouse monoclonal hybridoma clones are described in and can be ordered from HyTest Ltd., Catalog ID 4C28.
- suitable antibodies capable for detecting interleukin-6, (clinical marker to validate status of inflammation), for example mouse monoclonal hybridoma clones B10, G5. These mouse monoclonal hybridoma clones are described in and can be ordered from HyTest Ltd., Catalog ID 4IL6..

The "molecule captured at the antibody binding site" may be identical with the afore described "target polypeptide", i.e. is selected from any polypeptide, peptide, substrate, antigen or analyte, against which the antibody comprised in the conjugate of the invention has been created, and which is capable of binding to the binding region of the antibody. More preferably, the molecule captured at the antibody binding site is an antigen or fragment thereof, which is capable of binding to the antibody binding region.

The molecule captured at the antibody binding site is suitably a derivate (e.g. small peptide, fragment etc.) derived from the intact, wildtype or full length analyte or antigen present in a sample. The molecule captured at the antibody binding site may further be modified in such a way (e.g. by point mutations) that the affinity for binding to the antibody is altered, in particular reduced, compared to the intact analyte. Thereby the capability of the analyte to displace the molecule captured at the antibody binding site is improved. Altering, i.e. lowering of the affinity of the molecule captured at the antibody binding site may also be achieved by any other method known in art, such as chemical modification by, e.g. biotinylation, glycosylation etc.

Exemplary antigens, which may be detected using the conjugate and methods of the invention, are for example clinical relevant biomarkers like:
- Troponin-I, a clinical marker to validate heart disorders;
- Troponin-T, aclinical marker to validate heart disorders;
- CRP, a clinical marker to validate status of inflammation;
- Procalcitonin, a clinical marker to validate status of inflammation;
- Interleukin-6, a clinical marker to validate status of inflammation;
- Interleukin-8, a clinical marker to validate status of inflammation
- Interleukin-11, a clinical marker to validate status of inflammation;
- Parathyroid Hormone, a clinical marker to monitor and validate Parathyroidectomy;
- D-dimer, a clinical marker to validate blood coagulation;
- NT-proBNP, a clinical marker to validate heart disorders;
- BNP, a clinical marker to validate heart disorders;
- Insulin, (clinical marker to validate metabolic syndrome;
- C-Peptide, a (clinical marker to validate metabolic syndrome; and
- Cystatin C, a clinical marker to validate kidney diseases.

The invention is, however, not limited to the aforementioned antigens. The conjugate and methods of the invention can be adapted to any known or so far unknown antigen, analyte or biomarker.

In another preferred embodiment, the molecule captured at the antibody binding site is a fragment of an antigen, which may be known in the art, or which is a synthetic peptide. The amino acid sequence of a synthetic peptide is suitably adapted such that the binding to the binding site of the antibody comprised in the conjugate of the invention is facilitated. In a more preferred embodiment, such antigen fragment or synthetic peptide has a chain length of 4 to 22 amino acids, more preferably of 5 to 15 amino acids, most preferably of 6 to 12 amino acids. This has the advantage that a specific intramolecular quenching only at the antigen binding site can be ensured.

The invention provides a novel conjugate for use in *Fluorescence Quenching Immunoassays.* Accordingly, in a further preferred embodiment of the invention, the molecule captured at the antibody binding site is labeled with a signal generating molecule.

The signal- generating molecule is suitably selected from the group consisting of: fluorescent labels, enzyme labels, radioisotopes, chemiluminescent labels, electrochemiluminescent labels, bioluminescent labels, polymers, polymer particles, quantum dots, metal particles, haptens, and dyes. In other particular embodiments, the signal generating moiety comprises an enzymatic label.

Preferred according to invention is a molecule captured at the antibody binding site, which is labeled with a fluorophore or chemiluminophore. Suitable fluorophores or chemiluminophores are in general commercially available from various sources. Preferred according to the invention are TRF dyes and quantum dots.

A "fluorophore" according to the invention is preferably selected from the general class known as cyanine dyes, with emission wavelengths between 550 nm and 900 nm. These dyes may contain methine groups and their number influences the spectral properties of the dye. The monomethine dyes that are pyridines typically have blue to blue-green fluorescence emission, while quinolines have green to yellow-green fluorescence emission. The trimethine dye analogs are substantially shifted toward red wavelengths, and the pentamethine dyes are shifted even further, often exhibiting infrared fluorescence emission. Fluorophores or chemiluminophores are in general commercially available from various sources. Preferred according to the invention are TRF dyes and quantum dots.

TRF (time-resolved fluorometry) dyes involve fluorophores that are based on lanthanide ion complexes. Lanthanide metals are particularly useful. Certain life science applications take advantage of the unique fluorescence properties of lanthanide ion complexes (Ln(III) chelates or cryptates). These are well-suited due to their large Stokes shifts and extremely long emission lifetimes (from microseconds to milliseconds) compared to more traditional fluorophores (e.g. fluorescein, allophycoyanin, phycoerythrin, and rhodamine).

The two most commonly used lanthanides in life science assays with their corresponding acceptor dye are Europium³⁺/Allophycocyanin and Terbium³⁺/ Phycoerythrin.

The advantage of using TRF dyes is because biological fluids or serum commonly used in these assays contain many compounds and proteins which are autofluorescent. Therefore, the use of conventional, steady-state fluorescence measurement presents serious limitations in assay sensitivity. Long-lived fluorophores, such as lanthanides, combined with time-resolved detection (a delay between excitation and emission detection) minimizes prompt fluorescence interference.

TRF dyes are especially useful in FRET assays.

Related dyes can be used in accordance with the invention and further selected from cyclobutenedione derivatives, substituted cephalosporin compounds, fluorinated squaraine compositions, symmetrical and unsymmetrical squaraines, alkylalkoxy squaraines, or squarylium compounds. Some of these dyes can fluoresce at near infrared as well as at infrared wavelengths that would effectively expand the range of emission spectra up to about 1,000 nm. In addition to squaraines, i.e., derived from squaric acid, hydrophobic dyes such as phthalocyanines and naphthalocyanines can also be selected to operate at longer wavelengths. Other classes of fluorophores include 3-Hydroxypyrene 5,8,10-Tri Sulfonic acid, 5-Hydroxy Tryptamine (5-HT), Acid Fuhsin, Acridine Orange, Acridine Red, Acridine Yellow, Acriflavin, AFA (Acriflavin Feulgen SITSA), Alizarin Complexon, Alizarin Red, Allophycocyanin, ACMA, Aminoactinomycin D, Aminocoumarin, Anthroyl Stearate, Aryl- or Heteroaryl-substituted Polyolefin, Astrazon Brilliant Red 4 G, Astrazon Orange R, Astrazon Red 6B, Astrazon Yellow 7 GLL, Atabrine, Auramine, Aurophosphine, Aurophosphine G, BAO 9 (Bisaminophenyloxadiazole), BCECF, Berberine Sulphate, Bisbenzamide, BOBO 1, Blancophor FFG Solution, Blancophor SV, Bodipy F1, BOPRO 1, Brilliant Sulphoflavin FF, Calcien Blue, Calcium Green, Calcofluor RW Solution, Calcofluor White, Calcophor White ABT Solution, Calcophor White Standard Solution, Carbocyanine, Carbostyryl, Cascade Blue, Cascade Yellow, Catecholamine, Chinacrine, Coriphosphine O, Coumarin, Coumarin-Phalloidin, CY3.1 8, CY5.1 8, CY7, Dans (1-Dimethyl Amino Naphaline 5 Sulphonic Acid), Dansa (Diamino Naphtyl Sulphonic Acid), Dansyl NH-CH3, DAPI, Diamino Phenyl Oxydiazole (DAO), Dimethylamino-5-Sulphonic acid, Dipyrrometheneboron Difluoride, Diphenyl Brilliant Flavine 7GFF, Dopamine, Eosin, Erythrosin ITC, Ethidium Bromide, Euchrysin, FIF (Formaldehyde Induced Fluorescence), Flazo Orange, Fluo 3, Fluorescamine, Fura-2, Genacryl Brilliant Red B, Genacryl Brilliant Yellow 10GF, Genacryl Pink 3G, Genacryl Yellow 5GF, Gloxalic Acid, Granular Blue, Haematoporphyrin, Hoechst 33258, Indo-1, Intrawhite Cf Liquid, Leucophor PAF, Leucophor SF, Leucophor WS, Lissamine Rhodamine B200 (RD200), Lucifer Yellow CH, Lucifer Yellow VS, Magdala Red, Marina Blue, Maxilon Brilliant Flavin 10 GFF, Maxilon Brilliant Flavin 8 GFF, MPS (Methyl Green Pyronine Stilbene), Mithramycin, NBD Amine, Nile Red, Nitrobenzoxadidole, Noradrenaline, Nuclear Fast Red, Nuclear Yellow, Nylosan Brilliant Flavin E8G, Oregon Green, Oxazine, Oxazole, Oxadiazole, Pacific Blue, Pararosaniline (Feulgen), Phorwite AR Solution, Phorwite BKL, Phorwite Rev, Phorwite RPA, Phosphine 3R, Phthalocyanine, Phycoerythrin R, Polyazaindacene Pontochrome Blue Black, Porphyrin, Primuline, Procion Yellow, Propidium Iodide, Pyronine, Pyronine B, Pyrozal Brilliant Flavin 7GF, Quinacrine Mustard, Rhodamine 123, Rhodamine 5 GLD, Rhodamine 6G, Rhodamine B, Rhodamine B 200, Rhodamine B Extra, Rhodamine BB, Rhodamine BG, Rhodamine WT, Rose Bengal, Serotonin, Sevron Brilliant Red 2B, Sevron Brilliant Red 4G, Sevron Brilliant Red B, Sevron Orange, Sevron Yellow L, SITS (Primuline), SITS (Stilbene Isothiosulphonic acid), Stilbene, Snarf 1, sulpho Rhodamine B Can C, Sulpho Rhodamine G Extra, Tetracycline, Texas Red, Thiazine Red R, Thioflavin S, Thioflavin TCN, Thioflavin 5, Thiolyte, Thiozol Orange, Tinopol CBS, TOTO 1, TOTO 3, True Blue, Ultralite, Uranine B, Uvitex SFC, Xylene Orange, XRITC, YO PRO 1, or combinations thereof.

One skilled in the art would know which one to select among such fluorescence dyes as long as the desired emission and absorption properties as well as their chemical, such as hydrophobic properties are appropriate. The spectral properties of the fluorescent dyes should, according to a preferred embodiment of the invention, be sufficiently similar in excitation wavelengths and intensity to fluorescein or rhodamine derivatives as to permit the use of the same.

Attaching of the fluorophore to the molecule, which is captured or which is to be captured at the binding site of the antibody which is comprised in the conjugate of the invention, may be achieved by any of the techniques familiar to those skilled in the art. For example, the fluorophore may be covalently attached to the biomolecular probe by methods disclosed in US 5,194,300 and US 4,774,189.

In a further preferred embodiment of the invention, the fluorophore facilitates quenching, in particular dark quenching in combination with a quencher molecule or dark quencher molecule, which is attached to the linker.

Accordingly, the invention further provides a conjugate, wherein the linker comprises a quencher or functions as quencher molecule. More preferably, the linker comprises a dark quencher.

Fluorescence Resonance Energy Transfer (FRET) is a process whereby a first fluorescent dye (the "donor" dye) is excited, typically by illumination, and transfers its absorbed energy to a second dye (the "acceptor" dye) that has a longer wavelength and therefore lower energy emission. Where the second dye is fluorescent, energy transfer results in fluorescence emission at the wavelength of the second dye. However, where the second dye is nonfluorescent, the absorbed energy does not result in fluorescence emission, and the fluorescence of the initial donor dye is said to be "quenched". Energy transfer can also be utilized to quench the emission of luminescent donors, including phosphorescent and chemiluminescent donors. When a luminescent emission is restored by preventing energy transfer, the luminescence is said to be "dequenched" or "unquenched". The use of a variety of dyes to quench fluorescence is known in the art. The application of this phenomenon to analyze biological systems is also well-detailed. FRET has been utilized to study DNA hybridization and amplification, the dynamics of protein folding, proteolytic degradation, and interactions between other biomolecules.

A quencher may itself be a fluorescent molecule which emits fluorescence at a characteristic wavelength. Thus, a fluorophore may act as a quencher when appropriately coupled to another dye and vice versa. In this case, increase in fluorescence from the acceptor molecule, which is of a different wavelength to that of the donor label, will also indicate binding of the antibody binding site. Alternatively, the acceptor does not fluoresce ("dark acceptor" or "dark quencher"): Such acceptors include DABCYL, methyl red, QSY-7 diarylrhodamine dyes and 6-(dimethylamino)-2-[4-[4 (dimethylamino)phenyl]- 1 ,3-butadienyl]- 1 -ethyl quinolinium perchlorate (CAS number 181885-68-7). Typical fluorophore/quencher compounds include certain rhodamine dyes or Cy5.

DABCYL (4'dimethylaminophenylazo) benzoic acid is a common dark quencher used widely in many assays, such as "molecular beacons" for DNA detection (US 5,989,823). Diazo dyes of the BHQ series, which are referred to as "Black Hole Quenchers" (WO 01/86001), provide a broad range of absorption which overlaps well with the emission of many fluorophores. The QSY series dyes from Molecular Probes are another series of dark quenchers used extensively as quenching reagents in many bioassays (US 6,399,392). The structure of QSY 7 is illustrated in US 2005/0014160. QSY-7 is a nonfluorescent diarylrhodamine derivative. QSY21 is a nonfluorescent diarylrhodamine chromophore with strong absorption in the visible spectrum, and is an effective fluorescence quencher.

By far the most common donor-acceptor dye pair utilized in biomedical applications is DABCYL (the quenching dye) and EDANS (the fluorophore).

A preferred dark quencher - fluorophore pair according to the invention is selected from Atto612Q (Kokko, T. et al., 2007) and DyQ2 (Dyomics GmbH, Germany; MW: 842.88 g/mol; *Molecular formula:* C40H39N2O13S2 * Na) (dark quencher) and EuLH (as the fluorophore). EuLH is (6,9-dicarboxymethyl-3-{4-([1,10]-phenanthrol-2-ylethinylphenyl-carbamoyl)-methyl}-3,6,9-triaza-)-undeca-1,11-dicarboxylic acid (LH4) in complex with Eu³⁺ (Zuchner et al., 2009).

The quencher, as referred to herein, is coupled to a linker. The linker is selected from a variety of linking groups, including lower alkyl, cycloalkyl and heterocycloalkyl linking groups that extend from the active quencher to the core structure, i.e. the Fc region of the antibody comprised in the conjugate of the invention, where the linker-quencher molecule is attached. Preferably, the quencher, more preferably the dark quencher, is attached to the distal end of the linker. Attached in this regard means that the dark quencher is bonded to the distal end of the linker via an amino-NHS ester group or a thiol-maleimide group.

In a preferred embodiment of the invention, the dark quencher is bonded to the distal end of the linker via an amino-NHS ester group.

In another embodiment of the invention, the dark quencher is bonded to the distal end of the linker via a thiol-maleimide group.

At its proximal end, the linker is bonded via a novel carbohydrate linker coupling (CLC) approach using an N-glycosylated asparagine residue in the Fc chain of the antibody comprised in the conjugate of the invention as chemical reactive group. Preferably, the N-glycosylated asparagine 297 on the Fc antibody fragment of the antibody comprised in the conjugate of the invention is used as chemical reactive group. Asparagine is naturally occurring in almost all antibodies, particularly in monoclonal antibodies at position 297 of the Fc chain. However, the scope of the invention also comprises engineered antibodies, in which, for example by point mutations, amino acid substitutions, deletions or insertions the position of the asparagine is moved to a position in close proximity to position 297. Close proximity means a position in the range of 294 to 300 of the Fc chain, preferably in the range of 295 to 299, more preferably in the range of 296 to 298. Since glycosylation in antibodies is specific to asparagine 297, it was surprisingly found that this specific site can be used as intramolecular landmark. This means that the distance between the landmark and the antigen binding site stays constant, even among different monoclonal antibodies (Mizuochi, Taniguchi, Shimizu, & Kobata, 1982; Weitzhandler et al., 1994; Wright et al., 1997). However to facilitate direct molecular interactions, distances in the low nm range (e.g. for FRET interactions: 2-9 nm) are required (Mizuochi et al., 1982; Weitzhandler et al., 1994; Wright et al., 1997). In order to facilitate those interactions, the linker between the terminal carbohydrate group and the dark quencher shows the following characteristics:
Generally, preferred linkers have from 5 to 100 bonds from end to end, preferably 20 to 40 bonds, and may be branched or straight chain or contain rings. The bonds may be carbon-carbon or carbon-heteroatom or heteroatom-heteroatom bonds. The linkage can be designed to be hydrophobic or hydrophilic. The linker can contain single and/or double bonds, a number of 0 - 10 heteroatoms (O, S preferred), and saturated or aromatic rings. The linker may contain groupings such as ester, ether, sulfide, disulfide and the like.

In a most preferred embodiment, the length of the linker is adaptable such that the quencher molecule of the linker is interacting intramolecularly with the fluorophore of the molecule captured at the antibody binding site, thereby the fluorescence of the fluorophore being inhibited (quenched) by this binding.

In order to ensure a specific intramolecular quenching only at the antigen binding site in the conjugate of the invention, the linker has typically a length in the range of 5 to 85 Å, 10 to 65 Å, preferably in the range of 15 to 45 Å, more preferably in the range of 15 to 40 or 15 to 35 Å, most preferably in the range of 15 to 30, 20 to 30 or 20 to 25 Å. Best quenching results could be achieved with a linker length between 20 and 30 Å, particularly with a linker length of 25 Å.

In a preferred embodiment, the linker of the invention is a molecule according to formula (I):

A-L1-B-C-L2-D-E (I),

wherein
A is hydrazide, aminooxy or thiosemicarbazide;
L1 and L2 are selected from a bond, alkyl, polyethylene glycol (PEG), polyamide, peptide, carbohydrate, oligonucleotide or polynucleotide;
B is alkyne, azide, thiol, tetrazine, DBCO, TCO, vinyl or methylcyclopropene;
C is azide, alkyne, maleimide, TCO, vinyl, methylcyclopropene, azide or tetrazine;
D is a group selected from amino and thiol; and
E is a quencher, preferably a dark quencher as described herein, further comprising a NHS-ester group or a maleimide group, as described herein,

In a more preferred embodiment of the invention, the linker is a molecule according to formula (I) with the prerequisites that:
when B is alkyne, then C is azide;
when B is azide, then C is alkyne;
when B is thiol, then C is maleimide;
when B is tetrazine, then C is TCO, vinyl or methylcyclopropene;
when B is DBCO, then C is azide; and
when B is TCO, vinyl or methylcyclopropene, then C is tetrazine.

Chemical group A is bonded to the proximal end of the linker and connects the linker to the carbohydrate chain of the N-glycosylation site asparagine at position 297 or a position in close proximity to position 297 of the Fc chain of the antibody comprised in the conjugate of the invention.

Chemical groups B and C are reacted and bonded via click chemistry.

Chemical group D is bonded at the distal end of the linker and connects the distal end of the linker to the quencher molecule E.

The compounds of chemical group A may be combined and used with any compound of chemical group B.

Chemical groups A and B may form together a hydrazide alkyne group. Preferably, the hydrazine alkyne group formed by chemical groups A and B is a compound of formula (II):

H₂N-NH-(C=O)-L 1-C≡CH (II),

wherein
L1 is a bond or C₁₋₁₀alkyl.

Preferably, L1 is C₂₋₇alkyl.
More preferably, L1 is ethyl, propyl or butyl.
Most preferably, L1 is ethyl.

The compounds of formula (II) of the invention are suitably known in the art. Preferred compounds of formula (II) have an assigned CAS registry and/or ReaxysFile number and are selected from the compounds shown in Table 1.

**Table 1: Hydrazide-alkyne compounds of formula (II)**

| **Name** | **CAS- and ReaxysFile-number** | **Structure** |
|---|---|---|
| 4-Pentynoic acid, hydrazide | 114578-39-1 | 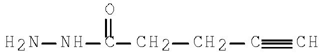 |
| | 4953267 | |
| 5-Hexynoic acid, hydrazide | 4230-19-7 | 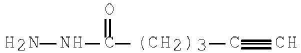 |
| | 4305355 | |
| 6-Heptynoic acid, hydrazide | 130905-57-6 | 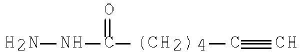 |
| | 4305493 | |
| 9-Decynoic acid, hydrazide | 4230-18-6 | 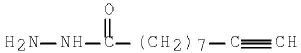 |

L1 and /or L2 may comprise, essentially consist of or consist of C₁₋₁₀alkyl, polyethylene glycol (PEG), a polyamide, a peptide, a carbohydrate, an oligonucleotide or a polynucleotide.

In a preferred embodiment according to the invention, the proximal end of the linker comprises, essentially consists of or consists of a compound of formula (II) and L2 is PEG.

Further preferably, L1 and L2 may both be PEG.
Further preferably, L1 is C₁₋₁₀alkyl and L2 is PEG.
Further preferably, L1 is PEG and L2 is C₁₋₁₀alkyl.

PEG is an oligomer or polymer composed of ethylene oxide monomers. Because different applications require different polymer chain lengths, PEGs are prepared by polymerization of ethylene oxide and are commercially available over a wide range of molecular weights from 300 g/mol to 10,000,000 g/mol. While PEGs with different molecular weights find use in different applications, and have different physical properties (e.g. viscosity) due to chain length effects, their chemical properties are nearly identical. Different forms of PEG are also available, depending on the initiator used for the polymerization process - the most common initiator is a monofunctional methyl ether PEG, or methoxypoly(ethylene glycol), abbreviated mPEG. Lower-molecular-weight PEGs are also available as purer oligomers, referred to as monodisperse, uniform, or discrete.

PEGs are also available with different geometries:
- Linear PEGs, where the ethylene oxide monomers are bound to each other in an unbranched polymer chain;
- Branched PEGs, which have three to ten PEG chains emanating from a central core group;
- Star PEGs, which have 10 to 100 PEG chains emanating from a central core group; and
- Comb PEGs, which have multiple PEG chains normally grafted onto a polymer backbone.

The numbers that are often included in the names of PEGs indicate their average molecular weights (e.g. a PEG with n = 9 would have an average molecular weight of approximately 400 daltons, and would be labeled PEG 400). Most PEGs include molecules with a distribution of molecular weights (i.e. they are polydisperse). The size distribution can be characterized statistically by its weight average molecular weight (Mw) and its number average molecular weight (Mn), the ratio of which is called the polydispersity index (Mw/Mn). Mw and Mn can be measured by mass spectrometry.

PEG is soluble in water, methanol, ethanol, acetonitrile, benzene, and dichloromethane, and is insoluble in diethyl ether and hexane.

In a preferred embodiment, the linker of the invention comprises a linear PEG. Using linear PEGs has the advantage that linear PEGs are cheap and possess a narrower molecular weight distribution.

When linear PEG is used to form the linker of the conjugate of the invention, it has suitably a molecular weight in the range of 40 Da to 1000 Da, preferably in the range of 100 Da to 800 Da and 150 Da to 600 Da, more preferably in the range of 200 Da to 400 Da, most preferably in the range of 200 Da to 300 Da or 220 Da to 310 Da. Even most preferably, the linear PEG comprised in the in the linker according to the invention has a molecular weight selected from 176 Da, 220 Da, 264 Da, 308 Da and 352 Da.

In another preferred embodiment, the linear PEG used to form the linker conjugate of the invention suitably consists of 1 to 20, preferably 2 to 15 or 3 to 10, more preferably 4 to 9 or 4 to 8, most preferably 5 to 7 ethylene oxide monomers. Further preferably, the linear PEG comprised in the linker according to the invention consists of 3, 4, 5, 6, 7, 8, 9, or 10, more preferably 4, 5, 6, 7, or 8, most preferably 5, 6 or 7 ethylene oxide monomers.

It should be noted that the entire linker (formula (I) as described herein) has a length in the range of 5 to 85 Å, 10 to 65 Å, preferably in the range of 15 to 45 Å, more preferably in the range of 15 to 40 or 15 to 35 Å, most preferably in the range of 15 to 30, 20 to 30 or 20 to 25 Å. Best quenching results could be achieved with a linker length between 20 and 30 Å, particularly with a linker length of 25 Å. Accordingly, the numbers of ethylene monomers for L1 and L2 together is to be in the above discussed range. This ensures the specific intramolecular quenching according to the invention.

If one of L1 or L2 is for example a C₁₋₁₀alkyl, the number of ethylene oxide monomers in the other of L1 and L2 is to be reduced accordingly in order to assure that the specific intramolecular quenching can occur.

At the proximal end, the linker is covalently bonded to the glycosylated Fc region of the antibody comprised in the conjugate of the invention, preferably at the N-glycosylated asparagine at position 297 or a position in close proximity to position 297. The bonding of the proximal end of the linker to the Fc region of the antibody is suitably performed via carbohydrate linker coupling, which is known in the art as "click chemistry".

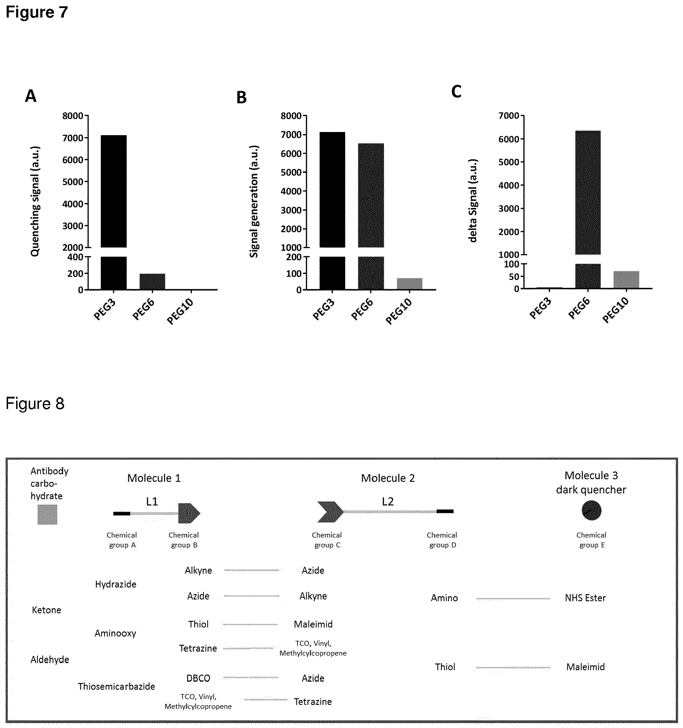

Suitably, bonding of the proximal end of the linker to the Fc region of the antibody is performed with a compound selected from
i. the group consisting of hydrazide-alkyne-azide, hydrazide-azide-alkyne, hydrazide-thiol-maleimide, hydrazide-DBCO-azide, hydrazide-TCO-tetrazine, hydrazide-methylcyclopropene-tetrazine and hydrazine-vinyl-tetrazine, wherein the hydrazide group of the linker is covalently bonded to an Fc region of the antibody;
ii. the group consisting of aminooxy-alkyne-azide, aminooxy-azide-alkyne, aminooxy-thiol-maleimide, aminooxy-DBCO-azide, aminooxy-TCO-tetrazine, aminooxy-methylcyclopropene-tetrazine and aminooxy-vinyl-tetrazine, wherein the aminooxy group of the linker is covalently bonded to an Fc region of the antibody; and
iii. the group consisting of thiosemicarbazide-alkyne-azide, thiosemicarbazide-azide-alkyne, thiosemicarbazide-thiol-maleimide, thiosemicarbazide-DBCO-azide, thiosemicarbazide-TCO-tetrazine, thiosemicarbazide-methylcyclopropene-tetrazine and thiosemicarbazide-vinyl-tetrazine, wherein the thiosemicarbazide group of the linker is covalently bonded to an Fc region of the antibody;
wherein said compounds of groups i. to iii. contain group L1 as shown in formula (I) herein and in figure 8.

DBCO refers to dibenzocyclooctyne and TCO refers to trans-cyclooctene.

Preferred according to the invention is the bonding of the proximal end of the linker to the Fc region of the antibody with a compound selected from group i. consisting of hydrazide-alkyne-azide, hydrazide-azide-alkyne, hydrazide-thiol-maleimide, hydrazide-DBCO-azide, hydrazide-TCO-tetrazine, hydrazide-methylcyclopropene-tetrazine and hydrazide-vinyl-tetrazine, wherein said compounds of group i. contain group L1 as shown in formula (I) herein and in figure 8 and wherein the hydrazide group of the linker is covalently bonded to an Fc region of the antibody.

Most preferred according to the invention is the bonding of the proximal end of the linker to the Fc region of the antibody via a hydrazide-alkyne-azide compound, wherein the hydrazide-alkyne compound is a compound of formula (II).

Further preferred according to the invention is the bonding of the proximal end of the linker to the Fc region of the antibody via a hydrazide-tetrazine-TCO compound.

Still further preferred according to the invention is the bonding of the proximal end of the linker to the Fc region of the antibody via an aminooxy-azide-alkyne compound.

In a preferred embodiment, the conjugate of the invention comprises, essentially consists of or consists of
- an antibody or fragment thereof,
- a linker of formula (I);
   o wherein said linker of formula (I) is bonded to the antibody at the proximal end via a compound selected from
      i. the group consisting of hydrazide-alkyne-azide, hydrazide-azide-alkyne, hydrazide-thiol-maleimide, hydrazide-DBCO-azide, hydrazide-TCO-tetrazine, hydrazide-methylcyclopropene-tetrazine and hydrazine-vinyl-tetrazine, wherein the hydrazide group of the linker is covalently bonded to an Fc region of the antibody;
      ii. the group consisting of aminooxy-alkyne-azide, aminooxy-azide-alkyne, aminooxy-thiol-maleimide, aminooxy-DBCO-azide, aminooxy-TCO-tetrazine, aminooxy-methylcyclopropene-tetrazine and aminooxy-vinyl-tetrazine, wherein the aminooxy group of the linker is covalently bonded to an Fc region of the antibody; and
      iii. the group consisting of thiosemicarbazide-alkyne-azide, thiosemicarbazide-azide-alkyne, thiosemicarbazide-thiol-maleimide, thiosemicarbazide-DBCO-azide, thiosemicarbazide-TCO-tetrazine, thiosemicarbazide-methylcyclopropene-tetrazine and thiosemicarbazide-vinyl-tetrazine, wherein the thiosemicarbazide group of the linker is covalently bonded to an Fc region of the antibody;
   o wherein said compounds of groups i. to iii. contain group L1 according to formula (I); and
   o wherein said linker comprises at the distal end a dark quencher selected from Atto612Q, DABCYL, methyl red, QSY-7 diarylrhodamine dyes and 6-(dimethylamino)-2-[4-[4 (dimethylamino)phenyl]- - 1 ,3-butadienyl]- 1 -ethyl quinolinium perchlorate, a rhodamine dye or Cy5;
- a molecule captured at the antibody binding site, which comprises a fluorophore.

In a more preferred embodiment, the conjugate of the invention comprises, essentially consists of or consists of
- a monoclonal antibody,
- a linker of formula (I);
   o wherein said linker is at the proximal end bonded to the Fc region of the antibody via a compound selected from:
      - hydrazide-alkyne-azide;
      - hydrazide-tetrazine-TCO; and
      - aminooxy-azide-alkyne;
   ∘ wherein L1 is PEG or C₁₋₁₀alkyl and L2 is PEG
   ∘ and wherein said linker comprises at the distal end the quencher Atto612Q;
- a molecule captured at the antibody binding site, which comprises the fluorophore EuLH.

In a most preferred embodiment, the conjugate of the invention comprises, essentially consists of or consists of
- a monoclonal antibody,
- a linker of formula (I);
   ∘ wherein said linker is at the proximal end bonded to the Fc region of the antibody via 4-pentynoic acid hydrazide; L2 is a liner PEG comprising 5 to 7, preferably 6 ethylene oxide monomers; and
   ∘ wherein said linker comprises at the distal end the dark quencher Atto612Q;
- a molecule captured at the antibody binding site, which comprises the fluorophore EuLH.

It should be noted that the compound 4-pentynoic acid hydrazide includes L1, which is ethyl.

Further preferably, the PEG containing linker has a length in the range of 15 to 45 Å, more preferably in the range of 15 to 40 or 15 to 35 Å, most preferably in the range of 15 to 30, 20 to 30 or 20 to 25 Å. Best quenching results could be achieved with a linker length between 20 and 30 Å, particularly with a linker length of 25 Å.

"Alkyne" as used herein means an unsaturated hydrocarbon containing at least one carbon-carbon triple bond. Preferred according to the invention are acyclic alkynes according to the general formula (III):

CₙH₂ₙ₋₂ (III)

wherein n is an integer between 3 to 12, preferably 4 to 9, more preferably 4 to 6, most preferably 4.

The quantity of linker molecules, preferably of linker molecules, such as linker molecules according to formula (I) comprised in the conjugate of the invention depends on the type of glycosylation. There are two heavy chains present in antibodies, i.e. the N-glycosylation site asparagine at position 297 or a position in close proximity to position 297 is present two times in the antibody. N-glycosylation of said asparagine may provide one or two carbohydrate chain ends, which are capable of binding linker molecules, such as linker molecules according to formula (I). Accordingly, in a preferred embodiment, the conjugate of the invention comprises between 2 to 4 linker molecules, most preferably 2 to 4 linker molecules of formula (I).

The invention further provides methods for preparing the conjugate of the invention.

The methods of the present invention use carbohydrates present on the Fc fragment of antibodies, particularly at the asparagine residue at position 297 or a position in close proximity to position 297, to site specifically introducing a uniform labeling chemistry (click chemistry).

After an oxidation reaction these carbohydrates are either present as aldehyde or ketone groups. By using a molecule such as alkyne hydrazide, it is possible without any enzymatic reactions to introduce for example an alkyne group on these carbohydrates. Alkyne groups can subsequently be used to specifically react with azide groups (click chemistry), which further allow efficient and specific labeling of antibodies with proteins or chemical components.

The reaction conditions to perform click chemistry reactions are well known in the art, as for example described in Presolski, Hong, & Finn, 2011.

A preferred method comprises the steps of
i. generating site specifically reactive aldehyde or ketone groups at the asparagine residue at position 297 or a position in close proximity to position 297 of the Fc region of the antibody by oxidizing the antibody;
ii. reacting the oxidized antibody with a compound A-L1-B, preferably selected from hydrazide-L1-alkyne, hydrazide-L1-azide, hydrazide-L1-thiol, hydrazide-L1-tetrazine, hydrazide-L1-DBCO, hydrazide-L1-TCO, hydrazide-L1-vinyl, hydrazide-L1-methylcyclopropene, aminooxy-L1-alkyne, aminooxy-L1-azide, aminooxy-L1-thiol, aminooxy-L1-tetrazine, aminooxy-L1-DBCO, aminooxy-L1-TCO, aminooxy-L1-vinyl, aminooxy-L1-methylcyclopropene, thiosemicarbazide-L1-alkyne, thiosemicarbazide-L1-azide, thiosemicarbazide-L1-thiol, thiosemicarbazide-L1-tetrazine, thiosemicarbazide-L1-DBCO, thiosemicarbazide-L1-TCO, thiosemicarbazide-L1-vinyl, thiosemicarbazide-L1-methylcyclopropene; wherein A, B and L1 are defined as described herein for the conjugate of the invention;
iii. preparing a compound C-L2-D-E; wherein C, L2, D and E are defined as herein described for the conjugate of the invention;
iv. reacting chemical group B of the antibody from step ii., which comprises the compound A-L1-B, with the C-L2-D-E compound of step iii. by click chemistry reactions; and
v. capturing a molecule which is capable of binding to the antibody binding site of the antibody and which comprises a signal generating molecule, such as a fluorophore.

It should be recognized that the advantages and advantageous embodiments described above for the conjugate according to the invention equally apply to the methods for preparing said conjugate such that it shall be referred to the above.

As for step i., the most preferred oxidant is p-periodate. Most studies used m-periodate which is the most water soluble of the periodate salts but difficult to solve at pH of 5.0 or higher (i.e., the pH range usually used in antibody oxidation). To overcome this problem p-periodate can be used. The preferred pH range for antibody oxidations using periodate is between 5.0 and 6.0. Preferably, periodate is used in a concentration between 10 mM - 50 mM. The reaction time should not exceed 1 hour.

As for step ii., aldehyde-activated (oxidized) sugars most preferably react with hydrazine or alkoxyamine at pH 5.0 -7.0. Aldehydes also react with primary amines to form Schiff bases which can be further reduced to form a covalent bond (reductive amination). Preferably, this reaction is catalysed by adding aniline in a 50-500 molar excess. The reaction time should not exceed 1 hour.

As for step iii., most preferably, the coupling of D-E is performed by reacting NHS-ester of chemical group E to primary amino groups of chemical group D. The reaction is performed between pH 8.0-9.2 using carbonate-bicarbonate, borate or phosphate buffer. Most preferably, the reaction is performed at 4 dC..

As for step iv., click reaction is most preferably performed by use of a Cu-THPTA catalyst at pH ranges between 7.0 and 7.6. Preferably, the concentration of the reaction components should be at least 10 µM. The reaction should react at room temperature not longer than 1 hour.

In a preferred embodiment, the oxidized antibody is reacted in step ii. of the method of the invention with a compound selected from hydrazide-L1-alkyne, hydrazide-L1-tetrazine or aminooxy-L1-azide.

In a further preferred embodiment, in step iv. of the method of the invention, a compound C-L2-D-E, wherein C is selected from azide, alkyne or TCO, is reacted with the oxidized antibody comprising the compound A-La1-B, wherein B is selected from alkyne, tetrazine or azide.

The invention further provides a conjugate obtainable by the afore described preparation method.

The conjugate of the invention is especially useful in diagnostic assays and methods. Thus, in a further embodiment, the invention provides the use of the conjugate of the invention in diagnostic assays.

The invention further relates to diagnostic assays and methods comprising the conjugate of the invention. Especially preferred are *in vitro* diagnostic methods.

Typically, an *in vitro* diagnostic method for the diagnosis of a disease or pathological condition comprises the following steps:
i. contacting a conjugate according to the invention with a sample from a subject suspected to be afflicted with a disease or condition to be diagnosed,
ii. detecting the amount of an analyte, antigen, biomarker or the like, or an isoform thereof, in said sample obtained from said subject;
iii. comparing the detected amount of said analyte, antigen or biomarker in said sample with an amount of the analyte, antigen or biomarker characteristic of a normal control;
whereby a changed amount of said analyte, antigen or biomarker in said sample relative to the normal control is a positive indicator of the disease or condition to be diagnosed.

"Changed amount" of the analyte, antigen or biomarker means either an elevated or a decreased amount of the analyte, antigen or biomarker. Preferred according to the invention is the detection of an elevated amount of the analyte, antigen or biomarker. However, there are conditions, in which the decrease of the amount of an analyte, antigen or biomarker leads to the development of a disease or pathological condition. The diagnostic method according to the invention is not limited to measure only elevated amounts of the analyte, antigen or biomarker. Accordingly, in an alternative embodiment, it is also preferred to measure a decreased amount of the analyte, antigen or biomarker.

The detection of the amount of an analyte, antigen, biomarker or the like, or an isoform thereof, in a sample is based according to invention by the replacement of the molecule captured at the antibody binding site of the antibody comprised in the conjugate of the invention by the analyte, antigen, biomarker or the like, or an isoform thereof, from the sample tested. Thereby, the captured molecule is released from the antibody binding site and the bond between the quencher molecule E of the linker of formula (I) and the fluorophore of the captured molecule is interrupted and "dequenched". Accordingly, dependent on the amount of the analyte, antigen or biomarker in the sample, a signal, preferably a fluorescence signal is generated and detected.

Moreover, the invention provides a simple method for detecting an analyte in a (biological) sample using the conjugate according to the invention, wherein the presence of the analyte in the sample induces or promotes the replacement of a captured molecule from the antigen binding site by the analyte, whereby a detectable signal is generated or changed.

The diagnostic method of the invention is also useful in monitoring the state of a disease or pathological condition, or to monitor the therapeutic effect of a medicament, based on measuring the amount of analyte, antigen or biomarker in a sample of a subject.

Said analyte, antigen or biomarker may be any analyte, antigen or biomarker which is capable of binding to the antibody binding site of the conjugate according to the invention. For example, the analyte, antigen or biomarker may be selected from:
- Troponin-I, a clinical marker to validate heart disorders;
- Troponin-T, a clinical marker to validate heart disorders;
- CRP, a clinical marker to validate status of inflammation;
- Procalcitonin, a clinical marker to validate status of inflammation;
- Interleukin-6, a clinical marker to validate status of inflammation;
- Interleukin-8, a clinical marker to validate status of inflammation
- Interleukin-11, a clinical marker to validate status of inflammation;
- Parathyroid Hormone, a clinical marker to monitor and validate Parathyroidectomy;
- D-dimer, a clinical marker to validate blood coagulation;
- NT-proBNP, a clinical marker to validate heart disorders;
- BNP, a clinical marker to validate heart disorders;
- Insulin, clinical marker to validate metabolic syndrome;
- C-Peptide, a clinical marker to validate metabolic syndrome; and
- Cystatin C, a clinical marker to validate kidney diseases.

Accordingly, the invention provides methods of diagnosing or monitoring the disease state of, e.g. diseases or conditions like heart disorders, inflammation, Parathyroidectomy, blood coagulation, metabolic syndrome and kidney diseases.

Heart disorders, which can be diagnosed using the conjugate of the invention, are for example cerebrovascular disease (e.g. stoke),ischemic heart disease (e.g. heart attack), hypertensive heart disease, rheumatic heart disease, inflammatory heart disease, congenital heart disease, cardiac arrhythmias and heart failure.

Inflammatory diseases, which can be diagnosed using the conjugate of the invention, are for example sepsis, Alzheimer's disease, ankylosing spondylitis, arthritis (osteoarthritis, rheumatoid arthritis (RA), psoriatic arthritis), asthma, atherosclerosis, Crohn's disease, colitis, dermatitis, diverticulitis, fibromyalgia, hepatitis, irritable bowel syndrome (IBS), systemic lupus erythematous (SLE), nephritis, Parkinson's disease and ulcerative colitis.

Blood coagulation conditions, which can be diagnosed using the conjugate of the invention, are for example disseminated intravascular coagulation and development of circulating anticoagulants.

Kidney diseases, which can be diagnosed using the conjugate of the invention, are for example Abderhalden-Kaufmann-Lignac syndrome (Nephropathic Cystinosis), Abdominal Compartment Syndrome, Acetaminophen-induced Nephrotoxicity, Acute Kidney Failure/Acute Kidney Injury, Acute Lobar Nephronia, Acute Phosphate Nephropathy, Acute Tubular Necrosis, Adenine Phosphoribosyltransferase Deficiency, Adenovirus Nephritis, Alagille Syndrome, Alport Syndrome, Amyloidosis, ANCA Vasculitis Related to Endocarditis and Other Infections, Angiomyolipoma, Analgesic Nephropathy, Anorexia Nervosa and Kidney Disease, Angiotensin Antibodies and Focal Segmental Glomerulosclerosis, Antiphospholipid Syndrome, Anti-TNF-a Therapy-related Glomerulonephritis, APOL1 Mutations, Apparent Mineralocorticoid Excess Syndrome, Aristolochic Acid Nephropathy, Chinese Herbal Nephropathy, Balkan Endemic Nephropathy, Bartter Syndrome, Beer Potomania, Beeturia, β-Thalassemia Renal Disease, Bile Cast Nephropathy, BK Polyoma Virus Nephropathy in the Native Kidney, Bladder Rupture, Bladder Sphincter Dyssynergia, Bladder Tamponade, Border-Crossers' Nephropathy, Bourbon Virus and Acute Kidney Injury, Burnt Sugarcane Harvesting and Acute Renal Dysfunction, Byetta and Renal Failure, C1q Nephropathy, Calcineurin Inhibitor Nephrotoxicity, Cannabinoid Hyperemesis Acute Renal Failure, Cardiorenal syndrome, Carfilzomib-Induced Renal Injury, CFHR5 nephropathy, Charcot-Marie-Tooth Disease with Glomerulopathy, Cherry Concentrate and Acute Kidney Injury, Cholesterol Emboli, Churg-Strauss syndrome, Chyluria, Cold Diuresis, Colistin Nephrotoxicity, Collagenofibrotic Glomerulopathy, Collapsing Glomerulopathy, Collapsing Glomerulopathy Related to CMV, Combination Antiretroviral (cART) Related-Nephropathy, Congenital Anomalies of the Kidney and Urinary Tract (CAKUT), Congenital Nephrotic Syndrome, Conorenal syndrome (Mainzer-Saldino Syndrome or Saldino-Mainzer Disease), Contrast Nephropathy, Copper Sulfate Intoxication, Cortical Necrosis, Crizotinib-related Acute Kidney Injury, Cryoglobuinemia, Crystalglobulin-Induced Nephropathy, Crystal-Induced Acute Kidney injury, Cystic Kidney Disease, Acquired, Cystinuria, Dasatinib-Induced Nephrotic-Range Proteinuria, Dense Deposit Disease (MPGN Type 2), Dent Disease (X-linked Recessive Nephrolithiasis), DHA Crystalline Nephropathy, Dialysis Disequilibrium Syndrome, Diabetes and Diabetic Kidney Disease, Diabetes Insipidus, Dietary Supplements and Renal Failure, Diffuse Mesangial Sclerosis, Diuresis, Down Syndrome and Kidney Disease, Drugs of Abuse and Kidney Disease, Duplicated Ureter, EAST syndrome, Ebola and the Kidney, Ectopic Kidney, Ectopic Ureter, Edema, Swelling, Erdheim-Chester Disease, Fabry's Disease, Familial Hypocalciuric Hypercalcemia, Fanconi Syndrome, Fraser syndrome, Fibronectin Glomerulopathy, Fibrillary Glomerulonephritis and Immunotactoid Glomerulopathy, Fraley syndrome, Focal Segmental Glomerulosclerosis, Focal Sclerosis, Focal Glomerulosclerosis, Galloway Mowat syndrome, Giant Cell (Temporal) Arteritis with Kidney Involvement, Gestational Hypertension, Gitelman Syndrome, Glomerular Diseases, Glomerular Tubular Reflux, Glycosuria, Goodpasture Syndrome, Hair Dye Ingestion and Acute Kidney Injury, Hantavirus Infection Podocytopathy, Heat Stress Nephropathy, Hematuria (Blood in Urine), Hemolytic Uremic Syndrome (HUS), Atypical Hemolytic Uremic Syndrome (aHUS), Hemophagocytic Syndrome, Hemorrhagic Cystitis, Hemorrhagic Fever with Renal Syndrome (HFRS, Hantavirus Renal Disease, Korean Hemorrhagic Fever, Epidemic Hemorrhagic Fever, Nephropathis Epidemica), Hemosiderinuria, Hemosiderosis related to Paroxysmal Nocturnal Hemoglobinuria and Hemolytic Anemia, Hepatic Glomerulopathy, Hepatic Veno-Occlusive Disease, Sinusoidal Obstruction Syndrome, Hepatitis C-Associated Renal Disease, Hepatorenal Syndrome, Herbal Supplements and Kidney Disease, High Blood Pressure and Kidney Disease, HIV-Associated Immune Complex Kidney Disease (HIVICK), HIV-Associated Nephropathy (HIVAN), HNF1B-related Autosomal Dominant Tubulointerstitial Kidney Disease, Horseshoe Kidney (Renal Fusion), Hunner's Ulcer, Hyperaldosteronism, Hypercalcemia, Hyperkalemia, Hypermagnesemia, Hypernatremia, Hyperoxaluria, Hyperphosphatemia, Hypocalcemia, Hypocomplementemic Urticarial Vasculitic Syndrome, Hypokalemia, Hypokalemia-induced renal dysfunction, Hypokalemic Periodic Paralysis, Hypomagnesemia, Hyponatremia, Hypophosphatemia, Hypophosphatemia in Users of Cannabis, Ifosfamide Nephrotoxicity, IgA Nephropathy, IgG4 Nephropathy, Immersion Diuresis, Immune-Checkpoint Therapy-Related Interstitial Nephritis, Interstitial Cystitis, Painful Bladder Syndrome (Questionnaire), Interstitial Nephritis, Ivemark's syndrome, Ketamine-Associated Bladder Dysfunction, Kidney Stones, Nephrolithiasis, Kombucha Tea Toxicity, Lead Nephropathy and Lead-Related Nephrotoxicity, Lecithin Cholesterol Acyltransferase Deficiency (LCAT Deficiency), Leptospirosis Renal Disease, Light Chain Deposition Disease, Monoclonal Immunoglobulin Deposition Disease, Liddle Syndrome, Lightwood-Albright Syndrome, Lipoprotein Glomerulopathy, Lithium Nephrotoxicity, LMX1B Mutations Cause Hereditary FSGS, Loin Pain Hematuria, Lupus, Systemic Lupus Erythematosis, Lupus Kidney Disease, Lupus Nephritis, Lupus Nephritis with Antineutrophil Cytoplasmic Antibody Seropositivity, Lupus Podocytopathy, Lyme Disease-Associated Glomerulonephritis, Lysinuric Protein Intolerance, Lysozyme Nephropathy, Malarial Nephropathy, Malignancy-Associated Renal Disease, Malignant Hypertension, Malakoplakia, MDMA (Molly; Ecstacy; 3,4-Methylenedioxymethamphetamine) and Kidney Failure, Meatal Stenosis, Medullary Cystic Kidney Disease, Urolodulin-Associated Nephropathy, Juvenile Hyperuricemic Nephropathy Type 1, Medullary Sponge Kidney, Megaureter, Melamine Toxicity and the Kidney, Membranoproliferative Glomerulonephritis, Membranous Nephropathy, Membranous-like Glomerulopathy with Masked IgG Kappa Deposits, MesoAmerican Nephropathy, Metabolic Acidosis, Metabolic Alkalosis, Methotrexate-related Renal Failure, Microscopic Polyangiitis, Milk-alkalai syndrome, Minimal Change Disease, Mouthwash Toxicity, MUC1 Nephropathy, Multicystic dysplastic kidney, Multiple Myeloma, Myeloproliferative Neoplasms and Glomerulopathy, Nail-patella Syndrome, Nephrocalcinosis, Nephrogenic Systemic Fibrosis, Nephroptosis (Floating Kidney, Renal Ptosis), Nephrotic Syndrome, Neurogenic Bladder, Nodular Glomerulosclerosis, Non-Gonococcal Urethritis, Nutcracker syndrome, Oligomeganephronia, Orofaciodigital Syndrome, Orotic Aciduria, Orthostatic Hypotension, Orthostatic Proteinuria, Osmotic Diuresis, Osmotic Nephrosis, Ovarian Hyperstimulation Syndrome, Oxalate Nephropathy, Page Kidney, Papillary Necrosis, Papillorenal Syndrome (Renal-Coloboma Syndrome, Isolated Renal Hypoplasia), Parvovirus B19 and the Kidney, the Peritoneal-Renal Syndrome, Posterior Urethral Valve, Post-infectious Glomerulonephritis, Post-streptococcal Glomerulonephritis, Post-Infectious Glomerulonephritis (IgA-Dominant), Mimicking IgA Nephropathy, Polyarteritis Nodosa, Polycystic Kidney Disease, Posterior Urethral Valves, Post-Obstructive Diuresis, Preeclampsia, Propofol infusion syndrome, Proliferative Glomerulonephritis with Monoclonal IgG Deposits (Nasr Disease), Propolis (Honeybee Resin) Related Renal Failure, Proteinuria (Protein in Urine), Pseudohyperaldosteronism, Pseudohypobicarbonatemia, Pseudohypoparathyroidism, Pulmonary-Renal Syndrome, Pyelonephritis (Kidney Infection), Pyonephrosis, Radiation Nephropathy, Ranolazine and the Kidney, Refeeding syndrome, Reflux Nephropathy, Rapidly Progressive Glomerulonephritis, Renal Abscess, Peripnephric Abscess, Renal Agenesis, Renal Arcuate Vein Microthrombi-Associated Acute Kidney Injury, Renal Artery Aneurysm, Renal Artery Stenosis, Renal Cell Cancer, Renal Cyst, Renal Hypouricemia with Exercise-induced Acute Renal Failure, Renal Infarction, Renal Osteodystrophy, Renal Tubular Acidosis, Renin Mutations and Autosomal Dominant Tubulointerstitial Kidney Disease, Renin Secreting Tumors (Juxtaglomerular Cell Tumor), Reset Osmostat, Retrocaval Ureter, Retroperitoneal Fibrosis, Rhabdomyolysis, Rhabdomyolysis related to Bariatric Surgery, Rheumatoid Arthritis-Associated Renal Disease, Sarcoidosis Renal Disease, Salt Wasting, Renal and Cerebral, Schistosomiasis and Glomerular Disease, Schimke immuno-osseous dysplasia, Scleroderma Renal Crisis, Serpentine Fibula-Polycystic Kidney Syndrome, Exner Syndrome, Sickle Cell Nephropathy, Silica Exposure and Chronic Kidney Disease, Sri Lankan Farmers' Kidney Disease, Sjögren's Syndrome and Renal Disease, Synthetic Cannabinoid Use and Acute Kidney Injury, Kidney Disease Following Hematopoietic Cell Transplantation, Kidney Disease Related to Stem Cell Transplantation, Tea and Toast Hyponatremia, Thin Basement Membrane Disease, Benign Familial Hematuria, Trigonitis, Tuberculosis, Genitourinary, Tuberous Sclerosis, Tubular Dysgenesis, Immune Complex Tubulointerstitial Nephritis Due to Autoantibodies to the Proximal Tubule Brush Border, Tumor Lysis Syndrome, Uremia, Uremic Optic Neuropathy, Ureteritis Cystica, Ureterocele, Urethral Caruncle, Urethral Stricture, Urinary Incontinence, Urinary Tract Infection, Urinary Tract Obstruction, Urogenital Fistula, Uromodulin-Associated Kidney Disease, Vancomycin-Associated Cast Nephropathy, Vasomotor Nephropathy, Vesicointestinal Fistula, Vesicoureteral Reflux, Volatile Anesthetics and Acute Kidney Injury, Von Hippel-Lindau Disease, Waldenstrom's Macroglobulinemic Glomerulonephritis, Warfarin-Related Nephropathy, Wasp Stings and Acute Kidney Injury, Wegener's Granulomatosis, Granulomatosis with Polyangiitis, West Nile Virus and Chronic Kidney Disease, Wunderlich syndrome, Zellweger Syndrome, and Cerebrohepatorenal Syndrome.

The term "subject" refers to an animal, preferably a mammal, which is afflicted with, or suspected to be afflicted with a disease or pathological condition. Preferably, "subject" refers to a human. Accordingly, the conjugate of the invention is particularly suitable for use in the diagnosis of human diseases. Likewise, the conjugate of the invention is also suitable for use in veterinary medicine. The subject may for example be a pig, cattle, horse, sheep or poultry.

The term "sample" refers to any source of biological material, including, but not limited to, peripheral blood, plasma, lymphocytes, cerebrospinal fluid, urine, saliva, epithelia, fibroblasts, or any other sample comprising the analyte, antigen or biomarker.

In a preferred embodiment, the amount of the analyte, antigen or biomarker is detected in a body fluid sample obtained from a mammal, most preferably a human. The term "body fluid" refers to all fluids that are present in the human body including but not limited to blood, lymph, urine and cerebrospinal fluid (CSF). The blood sample may include a plasma sample or a serum sample, or fractions derived from these samples. The sample can be treated prior to use, such as preparing plasma from blood, diluting viscous fluids, and the like. Preferably, the plasma sample is treated with an anti-coagulant, such as EDTA.

According to a preferred embodiment of the present invention, the amount of the analyte, antigen or biomarker is detected in a blood sample taken from the subject, more preferably a plasma sample. Thus, the present invention preferably relates to a method as described above, comprising the steps of: obtaining a plasma sample from said subject; detecting the amount of the analyte, antigen or biomarker in the plasma sample; comparing the detected amount of the analyte, antigen or biomarker in the plasma sample with the amount of the analyte, antigen or biomarker in a plasma sample from a normal control, whereby an elevated amount of the analyte, antigen or biomarker relative to the normal control is a positive indication of the disease or condition to be diagnosed. Elevated amounts of analytes, antigens or biomarkers have been shown to correlate with and are useful in aiding the diagnosis of diseases or conditions.

An "elevated amount" of an analyte, antigen or biomarker (or an isoform thereof) means that the amount of the analyte, antigen or biomarker detected in the samples of the subjects is greater than the mean amount of analyte, antigen or biomarker characteristic of a normal control subject beyond the range of experimental error, as known in the art. Preferably, the amount of analyte, antigen or biomarker detected in the samples of the subjects is 10 % greater than said mean amount of the analyte, antigen or biomarker characteristic of a normal control person. More preferably, the amount of analyte, antigen or biomarker (or an isoform thereof) detected in the samples of the subjects is 25 % greater, or, even more preferred 50 % or 75 % greater than said mean amount of analyte, antigen or biomarker characteristic of a normal control person. Most preferably, the amount of analyte, antigen or biomarker (or an isoform thereof) detected in the samples of the subjects is several times greater than said mean amount of analyte, antigen or biomarker characteristic of a normal control person, e.g. 2, 3, 4, 5, 6, 7, 8, 9, 10 or more times greater.

A "normal control" is a sample of the same type obtained from a subject, for example that is obtained from at least one normal control person or from the patient at another time, wherein said control person or subject is not afflicted with the disease or condition to be diagnosed. In an embodiment, the normal control is taken from the patient at an earlier time. One of skilled in the art will appreciate that the sample from the subject to be diagnosed is assessed against a normal control and that a significant elevation in the amount of the analyte, antigen or biomarker in the subject's sample is determined based on comparison to the controls used in the given assay.

The diagnostic assay of the invention can for example be performed in HTS format as a high throughput screen. For example, the diagnostic assay may be carried in a well plate, such as a 96- or 384-well plate, wherein each well will be loaded with a different sample and the conjugate of the invention. Each well plate may also contain samples from a normal control subject and positive controls. The diagnostic assay may also be performed on a microfluidic chip.

The invention further provides a diagnostic kit, comprising
- a compound A-L1-B, preferably selected from hydrazide-L1-alkyne, hydrazide-L1-azide, hydrazide-L1-thiol, hydrazide-L1-tetrazine, hydrazide-L1-DBCO, hydrazide-L1-TCO, hydrazide-L1-vinyl, hydrazide-L1-methylcyclopropene, aminooxy-L1-alkyne, aminooxy-L1-azide, aminooxy-L1-thiol, aminooxy-L1-tetrazine, aminooxy-L1-DBCO, aminooxy-L1-TCO, aminooxy-L1-vinyl, aminooxy-L1-methylcyclopropene, thiosemicarbazide-L1-alkyne, thiosemicarbazide-L1-azide, thiosemicarbazide-L1-thiol, thiosemicarbazide-L1-tetrazine, thiosemicarbazide-L1-DBCO, thiosemicarbazide-L1-TCO, thiosemicarbazide-L1-vinyl, thiosemicarbazide-L1-methylcyclopropene; wherein A, B and L1 are defined as described herein for the conjugate of the invention;
- a compound C-L2-D-E; wherein C, L2, D and E are defined as herein described for the conjugate of the invention;
- an oxidant for oxidizing an antibody,
- a fluorophore, and
- instructions for using said kit ingredients.

A suitable oxidant is for example sodium periodate.

Such a kit would enable a person skilled in the art to prepare a conjugate according to the invention using any known or unknown antibody.

In a more preferred embodiment, the kit of invention may comprise an already assembled conjugate of the invention, which has been designed against a particular analyte or biomarker and instructions for using the same.

In a most preferred embodiment, the kit of the invention enables a high throughput screen and comprises an already assembled conjugate of the invention, a well plate, such as a 96- or 384-well plate, wherein each well of the well plate is loaded the conjugate of the invention, and wherein said kit comprises negative and positive control samples.

Alternatively, the kit of the invention can comprise a microfluidic chip, which is used in the diagnostic method of invention using the conjugate of the invention.

The kit may further comprise additives such as stabilizers, positive and negative controls, and buffers (e.g. a block buffer) and the like. The relative amounts of the various reagents may be varied widely to provide for concentrations in solution of the reagents which substantially optimize the sensitivity of the assay. Particularly, the reagents may be provided as dry powders, usually lyophilized, including excipients which on dissolution will provide a reagent solution having the appropriate concentration.

The diagnostic kit of the invention is especially useful for the diagnosing and/or monitoring the disease state of, e.g. diseases or conditions like heart disorders, inflammation, Parathyroidectomy, blood coagulation, metabolic syndrome and kidney diseases as detailed above.

The conjugate, methods, uses and the kit described herein have several advantages, especially in regard to current state of the art labeling strategies. Homogeneous antibody conjugates comprising between 2-4 linker molecules of formula (I) per antibody (depending on glycosylation degree) can be produced, rather than inhomogeneous antibody conjugates comprising 0-8 labels per antibody. The affinity of the coupled antibodies is not influenced, but maintained. There occurs no hydrolysis and unwanted attachment of the linker molecules of formula (I) to other proteins. No enzymatic coupling strategies are necessary. The coupling reacting of the linker, which comprises the quencher molecule, to the antibody is a simple and fast chemical reaction rather than a complex two-step enzymatic reaction. Moreover, there is no need of antibody engineering. It is for example not necessary to express the antibody in an optimized cell cultures system, which ultimately leads to low antibody yields. There is further no need of glycoengineering. The labeling degree is independent of terminal carbohydrate species. Accordingly, the invention described herein provides a solution to generate discrete intramolecular antibody interactions. The conjugate, methods, uses and the kit described herein allow the quantification of biomarkers in the clinical relevant concentrations and contribute thereby to the prior art.

The invention is further described by 7 figures and a working example in more detail.
- Figure 1: shows schematically a Fluorescence Quenching Immunoassay known in the prior art. Competitive binding of a fluorophore labeled analyte. Applying the sample leads to a signal reduction, mediated by a shift of the steady state.
- Figure 2: shows Fluorescence Quenching Immunoassay by Kreisig et. al. The sample is mixed with a labeled peptide. Subsequently an antibody, conjugated with dark quenchers, is added, resulting into three reactive states: State 1 - the peptide is bound by the antibody - the fluorophore is in close distance to the dark quenchers and gets quenched. State 2 - the analyte binds the antibody, the peptide is in large distance (more than 9 nm) from the antibody and emits light. State 3 - the analyte binds the antibody, the peptide is in close distance (less than 9 nm) to the antibody and gets unspecified quenched by the dark quenchers. The overall steady state between the three states leads only to a minor signal change in equimolar concentrations.
- Figure 3: shows schematically a Fluorescence Quenching Displacement Immunoassay developed by Kreisig et. al. (Kreisig, Hoffmann, & Zuchner, 2013; Kreisig, Prasse, Zscharnack, Volke, & Zuchner, 2015) compared to the (B)

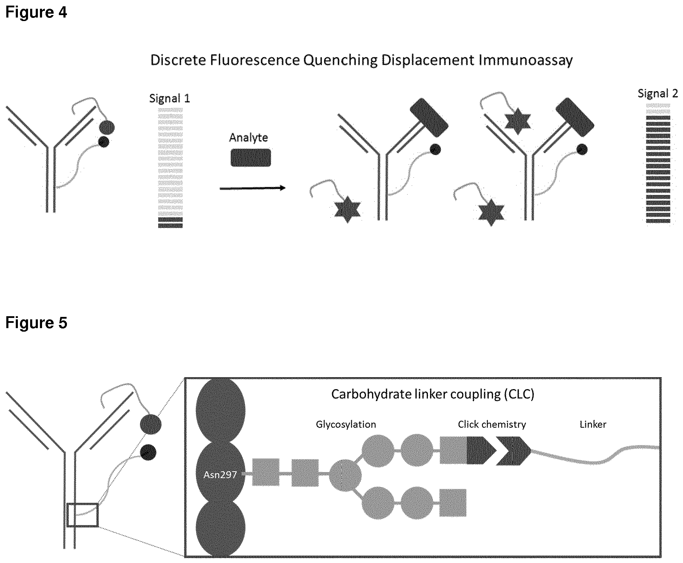

- Figure 4: shows schematically the Discrete Fluorescence quenching displacement Immunoassay according to the present invention.
- Figure 5: shows the schematic representation of our carbohydrate linker coupling (clc) strategy according to the invention.

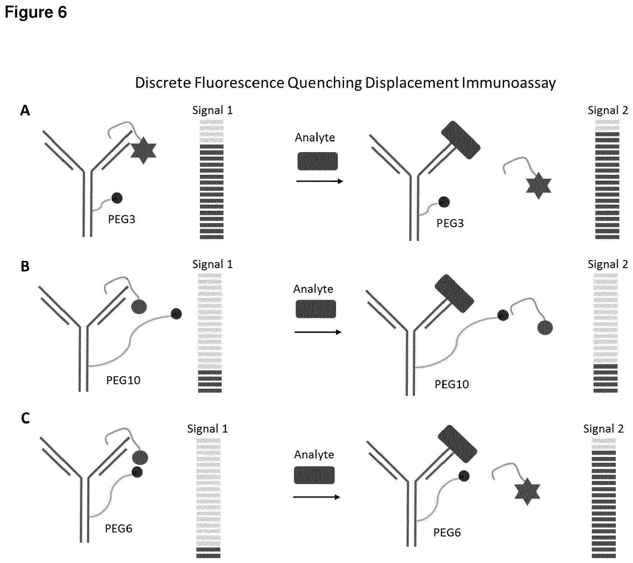

- Figure 6: shows the Discrete Fluorescence Quenching Displacement Immunoassay (DFQDI) according to the invention. The discrete labeled antibody and the peptide-fluorophore conjugate are incubated together. Consequently the fluorophore is in close distance to the dark quenchers and gets quenched. Subsequently the sample with unknown analyte concentration is applied to the peptide-antibody complex. Because the antibody-analyte complex has a greater affinity, compared to the antibody-peptide complex, the analyte is able to displace the peptide from the antigen binding site. As a result the peptide-fluorophore conjugate is in large distance (more than 9 nm) from the dark quencher and emits light. Thereby the delta signal (signal 2-signal 1) directly correlates with the analyte concentration in the applied sample. In order to reach a high assay sensitivity and a great dynamic range the delta signal should be maximal. Therefore the length of the conjugated linker is critical, both for signal quenching and signal generation after displacement. (A) The chosen linker length is to short, resulting into a low quenching efficiency. (B) The chosen linker length is too long, mainly resulting into unspecific fluorophore quenching. (C) The chosen linker length is optimal for both, quenching and signal generation.
- Figure 7: shows the influence of linker length (PEG3, PEG6 and PEG10) on Discrete Fluorescence Quenching Displacement Immunoassay. Measured fluorescence intensities (A) after incubating the dark quencher labeled antibody with the fluorophore labeled peptide, (B) after adding the analyte. (C) The shown delta signal is calculated by subtracting signal A from signal B.
- Figure 8: summarizes the strategies for the site-directed carbohydrate linker coupling of the invention.

### Example: 1 Synthesis of the Antibody-PEG10-dark Quencher conjugate

### 1.) Attachment of alkyne group on the antibody:

2 mg antibody where transferred into coupling buffer (0.1 M sodium phosphate, 0.15 M NaCl, pH 6.0). Sodium periodate was added until a final concentration of 50 mM was reached. The reaction was mix using a vortex mixer until the sodium periodate was completely dissolved and rotated for 40 min at room temperature with periodic mixing (20 rotations per minute). The reaction was stopped by adding 1% ethylene glycol and dialyzed against coupling buffer.

To conjugate an alkyne group alkyne hydrazine was added in a 50 fold molar access supported by a 1000 fold molar access of anilin. The reaction was incubated for 120 minutes at room temperature with constant mixing. The reaction was stopped by dialyzing against 100 mM potassium phosphate pH 6.8. The final antibody concentration has been determined using the Implen NP80-Touch Nano Photometer.

### 2.) Attachment of dark quencher on PEG-linker

10 mg of Azid-PEG-Amin linker where dissolved in 1 ml labeling buffer (20 Parts 1xPBS mixed with 1 Part 0.2 M sodium bicarbonate pH 9.0) and mixed with a 10 fold molar excess of dark quencher-NHS ester. The reaction was stopped after 1h and stored at -20 °C.

### 3.) Click reaction

The click reaction was generally performed as described (Presolski, Hong, & Finn, 2011).

In more detail, for a 2 ml reaction 25 µM alkyne from step 1 are mixed with 50 µM azide-PEG-quencher. Subsequently 30 µl of a 33 mM premixed THPTA-Cu complex (15 µl of a 20 mM CuSO4 solution mixed with 30 µl of a 50 mM THPTA solution) are added. Further 5 mM aminoguanidine (Stock 100 mM) and 5 mM sodium ascorbate (Stock 100 mM) are added. The reaction is rotated (20 rotations per minute) for 1 hour at room temperature. It is important to prevent the reaction from oxygen. Finally the reaction is dialyzed against a gradient of DMSO in PBS (5% DMSO in PBS, 2% DMSO in PBS, 1% DMSO in PBS and PBS).

### 4.) Adding the capture molecule

The discrete labeled antibody and the peptide-fluorophore conjugate are incubated together. Consequently, the fluorophore is in close distance to the dark quenchers and gets quenched.

### 5.) Discrete Fluorescence Quenching Displacement Immunoassay

Subsequently the sample with unknown analyte concentration is applied to the peptide-antibody complex. Because the antibody-analyte complex has a greater affinity, compared to the antibody-peptide complex, the analyte is able to displace the peptide from the antigen binding site. As a result the peptide-fluorophore conjugate is in large distance (more than 9 nm) from the dark quencher and emits light. Thereby the delta signal (signal 2-signal 1) directly correlates with the analyte concentration in the applied sample.
In order to reach a high assay sensitivity and a great dynamic range the delta signal should be maximal. Therefore the length of the conjugated linker is critical, both for signal quenching and signal generation after displacement. (A) The chosen linker length is to short, resulting into a low quenching efficiency. (B) The chosen linker length is too long, mainly resulting into unspecific fluorophore quenching. (C) The chosen linker length is optimal for both, quenching and signal generation (Figure 6).

It could be shown that a linker, with a discreet length, is essential to be introduced between the dark quencher and the carbohydrate groups, mainly because the interaction between the dark quencher and the fluorophore should be restricted to intramolecular interactions. Two aspects with equal significance have to be considered:
- First, the distance between the dark quencher and the fluorophore should be optimal for quenching. Meaning the linker should be long enough to bring the dark quencher in close spatial proximity. This parameter can be assessed by measuring the fluorescence signal emitted after the antibody and the peptide are premixed together.
- Secondly the linker should be as short as possible in order to reduce unspecific interactions between the dark quencher and free diffusing peptides. Meaning the linker should only allow intramolecular interactions but no intermolecular interactions. This parameter can be assessed by measuring the fluorescence signal emitted after the analyte is added.

The conjugate of the invention comprises an antibody with a linker molecule as described herein, which is usually covalently bonded to the antibody. The conjugate further comprises the peptide which is captured at the antibody binding site and optionally comprises the linker molecule. As such, the conjugate comprising these components is, in accordance with the invention, defined as one molecule. In this regard, the term "intramolecular interaction" means that the linker molecule intramolecular interacts with the peptide captured at the antibody binding site, either directly or via the fluorophore/quencher pairs as described herein.

Finally the optimal linker length can be identified by generating the delta signal (signal2-signal1). A high delta signal implies great signal to noise ratios and therefore an increased assay sensitivity (Figure 7).

The performance of three polyethylenglycol (PEG) linkers with a molecular length between 12.5 and 42 Å were tested and compared. All linkers where conjugated using a carbohydrate click chemistry approach and compared in equimolar 10 nM reactions. As dark quencher we used Atto612Q and EuLH (Zuchner et al., 2009) as fluorophore.

Using PEG3 linkers within the quenching experiment (premixed peptide and antibody), a high fluorescence signal (more than 6500 a.u.) was detected, whereas using PEG6 and PEG10 linkers less than 400 fluorescence units were detected (Figure 6a). This suggests that PEG3 linkers are not able to facilitate the, for the quenching process required, spatial proximity between quencher and fluorophore. In contrast the PEG6 and PEG10 linkers can generate the required spatial proximity.

To test whether the used linkers allow signal generation the analyte was added and measured again the fluorescence intensity. Conjugates with PEG3 and PEG6 linkers showed a high fluorescence signal (more than 6500 a.u.), whereas the conjugate with PEG10 linkers showed less than 200 fluorescence units (Figure 6b). This means that even in presence of an analyte the dark quenchers conjugated with PEG10 linkers are able to quench fluorescence signals from the peptides. Therefore it is likely that PEG10 linked dark quenchers are able to quench fluorescence intermolecular. Consequently the PEG10 linker is too long to only allow intramolecular interactions.

After calculating the delta signal (signal2-signal1) PEG6 linkers are the only tested linkers, which show a high delta fluorescence signal. This means that PEG6 linkers (with a length of 25 Å) are ideal to facilitate intramolecular interaction rather than intermolecular interactions and are thereby optimal for *Discrete Fluorescence Quenching Displacement Immunoassays.* PEG3, PEG6 and PEG10 refer to linear PEGs with 3, 6 and 10 ethylene oxide monomers, respectively.

### References

Agarwal, P., & Bertozzi, C. R. (2015). Site-Specific Antibody-Drug Conjugates: The Nexus of Bioorthogonal Chemistry, Protein Engineering, and Drug Development. Bioconjugate Chemistry, 26(2), 176-192. https://doi.org/10.1021/bc5004982
Chames, P., Van Regenmortel, M., Weiss, E., & Baty, D. (2009). Therapeutic antibodies: successes, limitations and hopes for the future. British Journal of Pharmacology, 157(2), 220-33. https://doi.org/10.1111/j.1476-5381.2009.00190.x
Clackson et al., Nature, 352:624-628 (1991)
DOVGAN, I. et al.: "Acyl Fluorides: Fast, Efficient, and Versatile Lysine-Based Protein Conjugation via Plug-and-Play Strategy", BIOCONJUGATE CHEMISTRY, (2017), 1452-1457
Ecker, D. M., Jones, S. D., & Levine, H. L. (2015). The therapeutic monoclonal antibody market. mAbs, 7(1), 9-14. https://doi.org/10.4161/19420862.2015.989042
FISCHER-DURAND N. et al.: "A new bioorthogonal cross-linker with alkyne and hydrazide end groups for chemoselective ligation. Application to antibody labelling", TETRAHEDRON, vol. 68, no. 47, (2012), 9638-9644
Hemmil, I., & Mukkala, V.-M. (2001). Time-Resolution in Fluorometry Technologies, Labels, and Applications in Bioanalytical Assays. Critical Reviews in Clinical Laboratory Sciences, 38(6), 441-519. https://doi.org/10.1080/20014091084254
Hollinger et al., Proc. Natl. Acad. Sol. USA, 90:6444-6448 (1993)
Jones et al., Nature, 321:522-525 (1986)
Kim, S., Ko, W., Park, H., & Lee, H. S. (2016). Efficient and Site-specific Antibody Labeling by Strain-promoted Azide-alkyne Cycloaddition. Journal of Visualized Experiments: JoVE, (118). https://doi.org/10.3791/54922
Köhler et al., Nature, 256:495 (1975)
Kreisig, T., Hoffmann, R., & Zuchner, T. (2011). Homogeneous fluorescence-based immunoassay detects antigens within 90 seconds. Analytical Chemistry, 83(11), 4281-4287. https://doi.org/10.1021/ac200777h
Kreisig, T., Hoffmann, R., & Zuchner, T. (2013). Highly Efficient F??rster Resonance Energy Transfer in a Fast, Serum-Compatible Immunoassay. ChemBioChem, 14(6), 699-702. https://doi.org/10.1002/cbic.201300073
Kreisig, T., Prasse, A. A., Zscharnack, K., Volke, D., & Zuchner, T. (2015). His-tag protein monitoring by a fast mix-and-measure immunoassay. Scientific Reports, 4(1), 5613. https://doi.org/10.1038/srep05613
Kokko, T., Kokko, L., Soukka, T., & Lövgren, L. Homogeneous non-competitive bioaffinity assay based on fluorescence resonance energy transfer, Anal. Chim. Acta 585, 120 (2007).
Kumar, A., Hao, G., Liu, L., Ramezani, S., Hsieh, J.-T., Oz, O. K., & Sun, X. (2015). Click-Chemistry Strategy for Labeling Antibodies with Copper-64 via a Cross-Bridged Tetraazamacrocyclic Chelator Scaffold. Bioconjugate Chemistry, 26(4), 782-789. https://doi.org/10.1021/acs.bioconjchem.5b00102
LE, H. T. et al.: "Antibody functionalization with a dual reactive hydrazide/click crosslinker", ANALYTICAL BIOCHEMISTRY, ELSEVIER, AMSTERDAM, NL, vol. 435, no. 1, (2013), 68-73
Leuvering, J. H. W., Thai, P. J. H. M., Waart, M. van der, & Schuurs, A. H. W. M. (1980). Sol Particle Immunoassay (SPIA). Journal of Immunoassay, 1(1), 77-91. https://doi.org/10.1080/01971528008055777
Liberatore, F. A., Comeau, R. D., McKearin, J. M., Pearson, D. A., Belonga, B. Q., Brocchini, S. J., ... Lawton, R. G. (1990). Site-directed chemical modification and crosslinking of a monoclonal antibody using equilibrium transfer alkylating crosslink reagents. Bioconjugate Chemistry, 1(1), 36-50. https://doi.org/10.1021/bc00001a005
LIU, G. et al.: "Doubly Caged Linker for AND-Type Fluorogenic Construction of Protein/Antibody Bioconjugates and In Situ Quantification", ANGEWANDTE CHEMIE INTERNATIONAL EDITION, vol. 56, no. 30, (2017), 8686-8691
Marks et al., J. Mol. Biol., 222:581-597 (1991)
Mizuochi, T., Taniguchi, T., Shimizu, A., & Kobata, A. (1982). Structural and numerical variations of the carbohydrate moiety of immunoglobulin G. The Journal of Immunology, 129(5). Retrieved from http://www.jimmunol.org/content/129/5/2016.long
Morgan, G., & Levinsky, R. (1985). Monoclonal antibodies in diagnosis and treatment. Archives of Disease in Childhood, 60, 96-98. Retrieved from http://adc.bmj.com/content/archdischild/60/2/96.full.pdf
Nargessi, R. D., Landon, J., & Smith, D. S. (1979a). Use of antibodies against the label in non-separation non-isotopic immunoassay: "Indirect quenching" fluoroimmunoassay of proteins. Journal of Immunological Methods, 26(4), 307-313. https://doi.org/10.1016/0022-1759(79)90176-5
Nargessi, R. D., Landon, J., & Smith, D. S. (1979b). Use of antibodies against the label in non-separation non-isotopic immunoassay: "Indirect quenching" fluoroimmunoassay of proteins. Journal of Immunological Methods, 26(4), 307-313. https://doi.org/10.1016/0022-1759(79)90176-5
Packard, B., Edidin, M., & Komoriya, A. (1986). Site-directed labeling of a monoclonal antibody: targeting to a disulfide bond. Biochemistry, 25(12), 3548-52. Retrieved from http://www.ncbi.nlm.nih.gov/pubmed/3718943
Pluckthun in The Pharmacology of Monoclonal Antibodies, vol. 113, Rosenburg and Moore eds., Springer-Verlag, New York, pp. 269-315 (1994)
Presolski, S. I., Hong, V. P., & Finn, M. G. (2011). Copper-Catalyzed Azide-Alkyne Click Chemistry for Bioconjugation. In Current Protocols in Chemical Biology. https://doi.org/10.1002/9780470559277.ch110148
Presta, Curr. Op. Struct. Biel., 2:593-596 (1992)
Reichmann et al, Nature. 332:323-329 (1988)
RESCHKE M. L. et al.: "Multifunctionalization of cetuximab with bioorthogonal chemistries and parallel EGFR profiling of cell-lines using imaging, FACS and immunoprecipitation approaches", BIOCHIMICA ET BIOPHYSICA ACTA (BBA) - PROTEINS & PROTEOMICS, ELSEVIER, NL, vol. 1844, no. 12, (2014), 2182-2192
Schumacher, D., Hackenberger, C. P. R., Leonhardt, H., & Helma, J. (2016). Current Status: Site-Specific Antibody Drug Conjugates. Journal of Clinical Immunology, 36 Suppl 1, 100-7. https://doi.org/10.1007/s10875-016-0265-6
Schumacher, D., Helma, J., Mann, F. A., Pichler, G., Natale, F., Krause, E., ... Leonhardt, H. (2015). Versatile and Efficient Site-Specific Protein Functionalization by Tubulin Tyrosine Ligase. Angewandte Chemie International Edition, 54(46), 13787-13791. https://doi.org/10.1002/anie.201505456
Scolnik, P. A. (2009). mAbs: a business perspective. mAbs, 1(2), 179-84. Retrieved from http://www.ncbi.nlm.nih.gov/pubmed/20061824
Siddiqui, M. Z. (2010). Monoclonal antibodies as diagnostics; an appraisal. Indian Journal of Pharmaceutical Sciences, 72(1), 12-7. https://doi.org/10.4103/0250-474X.62229
Sochaj, A. M., Swiderska, K. W., & Otlewski, J. (2015). Current methods for the synthesis of homogeneous antibody-drug conjugates. Biotechnology Advances, 33(6), 775-784. https://doi.org/10.1016/j.biotechadv.2015.05.001
Ullman, E. F., Schwarzberg, M., & Rubenstein, K. E. (1976). Fluorescent excitation transfer immunoassay. A general method for determination of antigens. The Journal of Biological Chemistry, 251(14), 4172-8. Retrieved from http://www.ncbi.nlm.nih.gov/pubmed/945272
Waldmann, T. A. (2003). Immunotherapy: past, present and future. Nature Medicine, 9(3), 269-277. https://doi.org/10.1038/nm0303-269
Weitzhandler, M., Hardy, M., Co, M. S., Avdalovic, N., Voragen, A. G. J., Bruggink, C., ... Arato, Y. (1994). Analysis of Carbohydrates on IgG Preparations?? Journal of Pharmaceutical Sciences, 83(12), 1670-1675. https://doi.org/10.1002/jps.2600831206
Wild, D. (2013). The immunoassay handbook: theory and applications of ligand binding, ELISA and related techniques*.*
Wright, A., Morrison, S. L., Shimizu, A., Kobata, A., Wilson, I. B. H., & Sim, R. B. (1997). Effect of glycosylation on antibody function: implications for genetic engineering. Trends in Biotechnology, 15(1), 26-32. https://doi.org/10.1016/S0167-7799(96)10062-7
Zimmerman, E. S., Heibeck, T. H., Gill, A., Li, X., Murray, C. J., Madlansacay, M. R., ... Sato, A. K. (2014). Production of Site-Specific Antibody-Drug Conjugates Using Optimized Non-Natural Amino Acids in a Cell-Free Expression System. Bioconjugate Chemistry, 25(2), 351-361. https://doi.org/10.1021/bc400490z
Zuchner, T., Schumer, F., Berger-Hoffmann, R., Müller, K., Lukas, M., Zeckert, K., ... Hoffmann, R. (2009). Highly sensitive protein detection based on lanthanide chelates with antenna ligands providing a linear range of five orders of magnitude. Analytical Chemistry, 81(22), 9449-9453. https://doi.org/10.1021/ac902175g
Zuk, R. F., Rowley, G. L., & Ullman, E. F. (1979). Fluorescence protection immunoassay: a new homogeneous assay technique. Clinical Chemistry, 25(9). Retrieved from http://clinchem.aaccjnls.org/content/25/9/1554.long

## Claims

1. A conjugate for fluorescence quenching immunoassays comprising an antibody bonded to a linker, **characterized in that** at its proximal end, the linker is bonded to the carbohydrate chain of the N-glycosylation site asparagine at position 297 or of the N-glycosylation site asparagine at a position in close proximity to position 297 of the Fc chain of the antibody the length of the linker is in the range 5 to 85 Å such that the distal terminus of the linker is capable of interacting with a molecule captured at the antigen binding site of the antibody; said linker is a molecule according to formula (I):
A-L1-B-C-L2-D-E (I),
wherein
A is hydrazide, aminooxy or thiosemicarbazide;
L1 and L2 are selected from a bond, C₁₋₁₀alkyl or polyethylene glycol (PEG);
B is alkyne, azide, thiol, tetrazine, DBCO, TCO, vinyl or methylcyclopropene;
C is azide, alkyne, maleimide, TCO, vinyl, methylcyclopropene, azide or tetrazine;
D is a group selected from amino and thiol; and
E is a dark quencher selected from Atto612Q, DABCYL, methyl red, QSY-7 diarylrhodamine dyes and 6- (dimethylamino)-2-[4-[4 (dimethylamino)phenyl]- - 1 ,3-butadienyl]- 1 -ethyl quinolinium perchlorate, a rhodamine dye or Cy5, further comprising a NHS-ester group or a maleimide group;
and said molecule captured at the antigen binding site of the antibody is an antigen or fragment thereof or a peptide, which is capable of binding to the antigen binding site of the antibody.

2. The conjugate of claim 1, wherein said molecule captured at the antigen binding site of the antibody is labeled, such as with a fluorophore or a signal generating molecule.

3. The conjugate according to claim 1 or 2, wherein:
when B is alkyne, then C is azide;
when B is azide, then C is alkyne;
when B is thiol, then C is maleimide;
when B is tetrazine, then C is TCO, vinyl or methylcyclopropene;
when B is DBCO, then C is azide; and
when B is TCO, vinyl or methylcyclopropene, then C is tetracine.

4. The conjugate of any of claims 1 to 3, wherein L1 and L2 are both PEG; or L1 is C₁₋₁₀alkyl and L2 is PEG; or L1 is PEG and L2 is C₁₋₁₀alkyl.

5. The conjugate of any of claims 1 to 3, wherein chemical groups A and B form together a hydrazide alkyne group, wherein said hydrazine alkyne group is a compound of formula (II):
H₂N-NH-(C=O)-L1-C≡CH (II),
wherein L1 is a bond or C₁₋₁₀alkyl.

6. The conjugate according to any one of the preceding claims, comprising, essentially consisting of or consisting of
• an antibody or fragment thereof,
• a linker of formula (I) as claimed in claim 1;
o wherein said linker of formula (I) is bonded to the antibody at the proximal end via a compound selected from
i. the group consisting of hydrazide-alkyne-azide, hydrazide-azide-alkyne, hydrazide-thiol-maleimide, hydrazide-DBCO-azide, hydrazide-TCO-tetrazine, hydrazide-methylcyclopropene-tetrazine and hydrazine-vinyl-tetrazine, wherein the hydrazide group of the linker is covalently bonded to an Fc region of the antibody;
ii. the group consisting of aminooxy-alkyne-azide, aminooxy-azide-alkyne, aminooxy-thiol-maleimide, aminooxy-DBCO-azide, aminooxy-TCO-tetrazine, aminooxy-methylcyclopropene-tetrazine and aminooxy-vinyl-tetrazine, wherein the aminooxy group of the linker is covalently bonded to an Fc region of the antibody; and
iii. the group consisting of thiosemicarbazide-alkyne-azide, thiosemicarbazide-azide-alkyne, thiosemicarbazide-thiol-maleimide, thiosemicarbazide-DBCO-azide, thiosemicarbazide-TCO-tetrazine, thiosemicarbazide-methylcyclopropene-tetrazine and thiosemicarbazide-vinyl-tetrazine, wherein the thiosemicarbazide group of the linker is covalently bonded to an Fc region of the antibody;
o wherein said compounds of groups i. to iii. contain group L1 according to formula (I) as defined in claim 1; and
o wherein said linker comprises at the distal end a dark quencher selected from Atto612Q, DABCYL, methyl red, QSY-7 diarylrhodamine dyes and 6-(dimethylamino)-2-[4-[4 (dimethylamino)phenyl]- - 1 ,3-butadienyl]- 1 -ethyl quinolinium perchlorate, a rhodamine dye or Cy5;
• a molecule captured at the antigen binding site of the antibody, which comprises a fluorophore.

7. The conjugate according to any one of the preceding claims, comprising, essentially consisting of or consisting of
• a monoclonal antibody,
• a linker of formula (I);
o wherein said linker is at the proximal end bonded to the Fc region of the antibody via a compound selected from:
• hydrazide-alkyne-azide;
• hydrazide-tetrazine-TCO; and
• aminooxy-azide-alkyne;
∘ wherein L1 is PEG or C₁₋₁₀alkyl and L2 is PEG
∘ and wherein said linker comprises at the distal end the quencher Atto612Q;
• a molecule captured at the antigen binding site of the antibody, which comprises the fluorophore EuLH.

8. The conjugate according to any one of the preceding claims, comprising, essentially consisting of or consisting of
• a monoclonal antibody,
• a linker of formula (I);
o wherein said linker is at the proximal end bonded to the Fc region of the antibody via 4-pentynoic acid hydrazide; L2 is a linear PEG comprising 5 to 7, preferably 6 ethylene oxide monomers; and
o wherein said linker comprises at the distal end the dark quencher Atto612Q;
• a molecule captured at the antigen binding site of the antibody, which comprises the fluorophore EuLH.

9. The conjugate of any one of the preceding claims, wherein the quenching with an analyte, antigen or biomarker is performed intramolecular.

10. A method for preparing the conjugate according to any one of claims 2 to 9, comprising the steps of:
i. generating site specifically reactive aldehyde or ketone groups at the asparagine residue at position 297 or a position in close proximity to position 297 of the Fc region of the antibody by oxidizing the antibody;
ii. reacting the oxidized antibody with a compound A-L1-B, preferably selected from hydrazide-L1-alkyne, hydrazide-L1-azide, hydrazide-L1-thiol, hydrazide-L1-tetrazine, hydrazide-L1-DBCO, hydrazide-L1-TCO, hydrazide-L1-vinyl, hydrazide-L1-methylcyclopropene, aminooxy-L1-alkyne, aminooxy-L1-azide, aminooxy-L1-thiol, aminooxy-L1-tetrazine, aminooxy-L1-DBCO, aminooxy-L1-TCO, aminooxy-L1-vinyl, aminooxy-L1-methylcyclopropene, thiosemicarbazide-L1-alkyne, thiosemicarbazide-L1-azide, thiosemicarbazide-L1-thiol, thiosemicarbazide-L1-tetrazine, thiosemicarbazide-L1-DBCO, thiosemicarbazide-L1-TCO, thiosemicarbazide-L1-vinyl, thiosemicarbazide-L1-methylcyclopropene; wherein A, B and L1 are as defined in claim 1;
iii. preparing a compound C-L2-D-E; wherein C, L2, D and E are as defined in claim 1;
iv. reacting chemical group B of the antibody from step ii., which comprises the compound A-L1-B, with the C-L2-D-E compound of step iii. by click chemistry reactions; and
v. capturing a molecule which is capable of binding to the antigen binding site of the antibody and which comprises a signal generating molecule, such as a fluorophore.

11. An *in vitro* method for the diagnosis of a disease or pathological condition comprising the steps of:
i. contacting a conjugate according to any one of claims 2 to 9 with a sample obtained from a subject suspected to be afflicted with a disease or condition to be diagnosed,
ii. detecting the amount of an analyte, antigen, biomarker or the like, or an isoform thereof, in said sample obtained from said subject;
iii. comparing the detected amount of said analyte, antigen or biomarker in said sample with an amount of the analyte, antigen or biomarker characteristic of a normal control;
whereby a changed amount, preferably an elevated amount of said analyte, antigen or biomarker in said sample relative to the normal control is a positive indicator of the disease or condition to be diagnosed.

12. A diagnostic kit, comprising
• a compound A-L1-B, preferably selected from hydrazide-L1-alkyne, hydrazide-L1-azide, hydrazide-L1-thiol, hydrazide-L1-tetrazine, hydrazide-L1-DBCO, hydrazide-L1-TCO, hydrazide-L1-vinyl, hydrazide-L1-methylcyclopropene, aminooxy-L1-alkyne, aminooxy-L1-azide, aminooxy-L1-thiol, aminooxy-L1-tetrazine, aminooxy-L1-DBCO, aminooxy-L1-TCO, aminooxy-L1-vinyl, aminooxy-L1-methylcyclopropene, thiosemicarbazide-L1-alkyne, thiosemicarbazide-L1-azide, thiosemicarbazide-L1-thiol, thiosemicarbazide-L1-tetrazine, thiosemicarbazide-L1-DBCO, thiosemicarbazide-L1-TCO, thiosemicarbazide-L1-vinyl, thiosemicarbazide-L1-methylcyclopropene; wherein A, B and L1 are as defined in claim 1;
• a compound C-L2-D-E; wherein C, L2, D and E are as defined in claim 2;
• an oxidant for oxidizing an antibody,
• a fluorophore, and
• instructions for using said kit ingredients for preparing and using the conjugate according to any one of claims 2 to 9.

13. A diagnostic kit comprising an already assembled conjugate as claimed in any one of claims 2 to 9, a well plate, such as a 96- or 384-well plate, or a microfluidic chip, wherein each well of the well plate or the microfluidic chip is loaded with the conjugate of the invention, and wherein said kit comprises negative and positive control samples.

14. The conjugate according to any one of claims 2 to 9 for use in diagnosing and/or monitoring the state of diseases or conditions like heart disorders, inflammation, Parathyroidectomy, blood coagulation, metabolic syndrome and kidney diseases.

## Patentansprüche

1. Konjugat für Fluoreszenzquenching-Immunassays, umfassend einen Antikörper, der an einen Linker gebunden ist, **dadurch gekennzeichnet, dass** an seinem proximalen Ende der Linker an die Kohlenhydratkette des N-Glykosylierungsstellen-Asparagins an Position 297 oder des N-Glykosylierungsstellen-Asparagins an einer Position in großer Nähe zu Position 297 der Fc-Kette des Antikörpers gebunden ist, die Länge des Linkers im Bereich von 5 bis 85 Å liegt, so dass der distale Terminus des Linkers mit einem an der Antigenbindungsstelle des Antikörpers eingefangenen Molekül interagieren kann, wobei der Linker ein Molekül gemäß der Formel (I) ist:
A-L1-B-C-L2-D-E (I),
wobei
A Hydrazid, Aminooxy oder Thiosemicarbazid ist;
L1 und L2 aus einer Bindung, C₁₋₁₀Alkyl oder Polyethylenglykol (PEG) ausgewählt sind;
B Alkin, Azid, Thiol, Tetrazin, DBCO, TCO, Vinyl oder Methylcyclopropen ist;
C Azid, Alkin, Maleinimid, TCO, Vinyl, Methylcyclopropen, Azid oder Tetrazin ist;
D eine aus Amino und Thiol ausgewählte Gruppe ist und
E ein Dunkelquencher ist, ausgewählt aus Atto612Q, DABCYL, Methylrot, QSY-7-Diarylrhodamin-Farbstoffen und 6-(Dimethylamino)-2-[4-[4-(dimethylamino)phenyl)]-1,3-butadienyl]-1-ethylchinoliniumperchlorat, einem Rhodaminfarbstoff oder Cy5, der ferner eine NHS-Estergruppe oder eine Maleinimidgruppe umfasst;
und wobei das an der Antigenbindungsstelle des Antikörpers eingefangene Molekül ein Antigen oder ein Fragment davon oder ein Peptid ist, das an die Antigenbindungsstelle des Antikörpers binden kann.

2. Konjugat nach Anspruch 1, wobei das an der Antigenbindungsstelle des Antikörpers eingefangene Molekül markiert ist, beispielsweise mit einem Fluorophor oder einem signalerzeugenden Molekül.

3. Konjugat nach Anspruch 1 oder 2, wobei:
wenn B Alkin ist, C dann Azid ist;
wenn B Azid ist, C dann Alkin ist;
wenn B Thiol ist, C dann Maleinimid ist;
wenn B Tetrazin ist, C dann TCO, Vinyl oder Methylcyclopropen ist;
wenn B DBCO ist, C dann Azid ist und
wenn B TCO, Vinyl oder Methylcyclopropen ist, C dann Tetrazin ist.

4. Konjugat nach einem der Ansprüche 1 bis 3, wobei L1 und L2 beide PEG sind oder L1 C₁₋₁₀Alkyl ist und L2 PEG ist oder L1 PEG ist und L2 C₁₋₁₀Alkyl ist.

5. Konjugat nach einem der Ansprüche 1 bis 3, wobei die chemischen Gruppen A und B zusammen eine Hydrazidalkingruppe bilden, wobei die Hydrazinalkingruppe eine Verbindung der Formel (II) ist:
H₂N-NH-(C=0)-L1-C≡CH (II),
wobei L1 eine Bindung oder C₁₋₁₀Alkyl ist.

6. Konjugat nach einem der vorhergehenden Ansprüche, umfassend, im Wesentlichen bestehend aus oder bestehend aus
• einen/einem Antikörper oder ein/einem Fragment davon,
• einen/einem Linker der Formel (I) wie in Anspruch 1 definiert;
∘ wobei der Linker der Formel (I) an den Antikörper am proximalen Ende über eine Verbindung gebunden ist, ausgewählt aus
i. der Gruppe bestehend aus Hydrazid-Alkin-Azid, Hydrazid-Azid-Alkin, Hydrazid-Thiol-Maleinimid, Hydrazid-DBCO-Azid, Hydrazid-TCO-Tetrazin, Hydrazid-Methylcyclopropen-Tetrazin und Hydrazid-Vinyl-Tetrazin, wobei die Hydrazid-Gruppe des Linkers kovalent an eine Fc-Region des Antikörpers gebunden ist;
ii. der Gruppe bestehend aus Aminooxy-Alkin-Azid, Aminooxy-Azid-Alkin, Aminooxy-Thiol-Maleinimid, Aminooxy-DBCO-Azid, Aminooxy-TCO-Tetrazin, Aminooxy-Methylcyclopropen-Tetrazin und Aminooxy-Vinyl-Tetrazin, wobei die Aminooxygruppe des Linkers kovalent an eine Fc-Region des Antikörpers gebunden ist; und
iii. der Gruppe bestehend aus Thiosemicarbazid-Alkin-Azid, Thiosemicarbazid-Azid-Alkin, Thiosemicarbazid-Thiol-Maleinimid, Thiosemicarbazid-DBCO-Azid, Thiosemicarbazid-TCO-Tetrazin, Thiosemicarbazid-Methylcyclopropen-Tetrazin, und Thiosemicarbazid-Vinyl-Tetrazin, wobei die Thiosemicarbazidgruppe des Linkers kovalent an eine Fc-Region des Antikörpers gebunden ist;
∘ wobei die Verbindungen der Gruppen i. bis iii. die Gruppe L1 gemäß Formel (I) wie in Anspruch 1 definiert enthalten; und
∘ wobei der Linker am distalen Ende einen Dunkelquencher umfasst, ausgewählt aus Atto612Q, DABCYL, Methylrot, QSY-7-Diarylrhodamin-Farbstoffen und 6-(Dimethylamino)-2-[4-[4-(dimethylamino)phenyl]-1,3-butadienyl]-1-ethylchinoliniumperchlorat, einem Rhodaminfarbstoff oder Cy5;
• ein/einem an der Antigenbindungsstelle des Antikörpers eingefangenes Molekül, das einen Fluorophor umfasst.

7. Konjugat nach einem der vorhergehenden Ansprüche, umfassend, im Wesentlichen bestehend aus oder bestehend aus
• einen/einem monoklonalen Antikörper,
• einen/einem Linker der Formel (I);
∘ wobei der Linker am proximalen Ende an die Fc-Region des Antikörpers über eine Verbindung gebunden ist, ausgewählt aus:
▪ Hydrazid-Alkin-Azid;
▪ Hydrazid-Tetrazin-TCO und
▪ Aminooxy-Azid-Alkin;
∘ wobei L1 PEG oder C₁₋₁₀Alkyl ist und L2 PEG ist
∘ und wobei der Linker am distalen Ende den Quencher Atto612Q umfasst;
• ein/einem an der Antigenbindungsstelle des Antikörpers eingefangenes Molekül, das den Fluorophor EuLH umfasst.

8. Konjugat nach einem der vorhergehenden Ansprüche, umfassend, im Wesentlichen bestehend aus oder bestehend aus
• einen/einem monoklonalen Antikörper,
• einen/einem Linker der Formel (I);
∘ wobei der Linker am proximalen Ende an die Fc-Region des Antikörpers über 4-Pentinsäurehydrazid gebunden ist; L2 ein lineares PEG ist, das 5 bis 7, vorzugsweise 6 Ethylenoxidmonomere umfasst und
∘ wobei der Linker am distalen Ende den Dunkelquencher Atto612Q umfasst;
• ein/einem an der Antigenbindungsstelle des Antikörpers eingefangenes Molekül, das den Fluorophor EuLH umfasst.

9. Konjugat nach einem der vorhergehenden Ansprüche, wobei das Quenchen mit einem Analyten, Antigen oder Biomarker intramolekular durchgeführt wird.

10. Verfahren zur Herstellung des Konjugats nach einem der Ansprüche 2 bis 9, umfassend die Schritte:
i. stellenspezifisches Erzeugen reaktiver Aldehyd- oder Ketongruppen am Asparaginrest an Position 297 oder einer Position in großer Nähe zu Position 297 der Fc-Region des Antikörpers durch Oxidieren des Antikörpers;
ii. Umsetzen des oxidierten Antikörpers mit einer Verbindung A-L1-B, die vorzugsweise ausgewählt ist aus Hydrazid-Ll-Alkin, Hydrazid-Ll-Azid, Hydrazid-Ll-Thiol, Hydrazid-Ll-Tetrazin, Hydrazid-Ll-DBCO, Hydrazid-Ll-TCO, Hydrazid-Ll-Vinyl, Hydrazid-Ll-Methylcyclopropen, Aminooxy-Ll-Alkin, Aminooxy-Ll-Azid, Aminooxy-Ll-Thiol, Aminooxy-Ll-Tetrazin, Aminooxy-Ll-DBCO, Aminooxy-Ll-TCO, Aminooxy-Ll-Vinyl, Aminooxy-Ll-Methylcyclopropen, Thiosemicarbazid-Ll-Alkin, Thiosemicarbazid-Ll-Azid, Thiosemicarbazid-Ll-Thiol, Thiosemicarbazid-Ll-Tetrazin, Thiosemicarbazid-Ll-DBCO, Thiosemicarbazid-Ll-TCO, Thiosemicarbazid-Ll-Vinyl, Thiosemicarbazid-Ll-Methylcyclopropen; wobei A, B und L1 wie in Anspruch 1 definiert sind;
iii. Herstellen einer Verbindung C-L2-D-E; wobei C, L2, D und E wie in Anspruch 1 definiert sind;
iv. Umsetzen der chemischen Gruppe B des Antikörpers aus Schritt ii., der die Verbindung A-L1-B umfasst, mit der C-L2-D-E-Verbindung von Schritt iii. durch Click-Chemie-Reaktionen und
v. Einfangen eines Moleküls, das an die Antigenbindungsstelle des Antikörpers binden kann und das ein signalerzeugendes Molekül, wie ein Fluorophor, umfasst.

11. *In vitro*-Verfahren zur Diagnose einer Krankheit oder eines pathologischen Zustands, umfassend die Schritte:
i. Inkontaktbringen eines Konjugats nach einem der Ansprüche 2 bis 9 mit einer Probe, die von einem Individuum erhalten wurde, von dem vermutet wird, dass es an einer zu diagnostizierenden Krankheit oder einem zu diagnostizierenden Zustand leidet,
ii. Nachweisen der Menge eines Analyten, Antigens, Biomarkers oder dergleichen oder einer Isoform davon in der von dem Individuum erhaltenen Probe;
iii. Vergleichen der nachgewiesenen Menge des Analyten, Antigens oder Biomarkers in der Probe mit einer Menge des Analyten, Antigens oder Biomarkers, die für eine normale Kontrolle charakteristisch ist,
wobei eine veränderte Menge, vorzugsweise eine erhöhte Menge, des Analyten, Antigens oder Biomarkers in der Probe im Vergleich zur normalen Kontrolle ein positiver Indikator für die zu diagnostizierende Krankheit oder den zu diagnostizierenden Zustand ist.

12. Diagnosekit, umfassend
• eine Verbindung A-L1-B, vorzugsweise ausgewählt aus Hydrazid-L1-Alkin, Hydrazid-Ll-Azid, Hydrazid-Ll-Thiol, Hydrazid-Ll-Tetrazin, Hydrazid-Ll-DBCO, Hydrazid-Ll-TCO, Hydrazid-Ll-Vinyl, Hydrazid-Ll-Methylcyclopropen, Aminooxy-Ll-Alkin, Aminooxy-Ll-Azid, Aminooxy-Ll-Thiol, Aminooxy-Ll-Tetrazin, Aminooxy-Ll-DBCO, Aminooxy-Ll-TCO, Aminooxy-Ll-Vinyl, Aminooxy-Ll-Methylcyclopropen, Thiosemicarbazid-Ll-Alkin, Thiosemicarbazid-Ll-Azid, Thiosemicarbazid-Ll-Thiol, Thiosemicarbazid-Ll-Tetrazin, Thiosemicarbazid-Ll-DBCO, Thiosemicarbazid-Ll-TCO, Thiosemicarbazid-Ll-Vinyl, Thiosemicarbazid-Ll-Methylcyclopropen; wobei A, B und L1 wie in Anspruch 1 definiert sind;
• eine Verbindung C-L2-D-E; wobei C, L2, D und E wie in Anspruch 2 definiert sind;
• ein Oxidationsmittel zum Oxidieren eines Antikörpers,
• einen Fluorophor und
• Anleitungen zur Verwendung der Kitbestandteile zur Herstellung und Verwendung des Konjugats nach einem der Ansprüche 2 bis 9.

13. Diagnosekit, umfassend ein bereits zusammengesetztes Konjugat nach einem der Ansprüche 2 bis 9, eine Platte mit Vertiefungen, wie eine Platte mit 96 oder 384 Vertiefungen, oder einen Mikrofluidik-Chip, wobei jede Vertiefung der Platte mit Vertiefungen oder der Mikrofluidik-Chip mit dem Konjugat der Erfindung beladen ist und wobei das Kit negative und positive Kontrollproben umfasst.

14. Konjugat nach einem der Ansprüche 2 bis 9 zur Verwendung bei der Diagnose und/oder Überwachung des Status von Krankheiten oder Zuständen wie Herzstörungen, Entzündung, Nebenschilddrüsenresektion, Blutgerinnung, metabolisches Syndrom und Nierenerkrankungen.

## Revendications

1. Conjugué pour immunoessais par extinction de fluorescence comprenant un anticorps lié à un lieur, **caractérisé en ce que**, à son extrémité proximale, le lieur est lié à la chaîne glucidique de l'asparagine de site de N-glycosylation à la position 297 ou de l'asparagine de site de N-glycosylation à une position étroitement proche de la position 297 de la chaîne Fc de l'anticorps, la longueur du lieur est dans la plage de 5 à 85 Å de sorte que l'extrémité distale du lieur puisse interagir avec une molécule capturée au site de liaison d'antigène de l'anticorps ; ledit lieur est une molécule selon la formule (I) :
A-L1-B-C-L2-D-E (I),
dans laquelle
A est hydrazide, aminooxy ou thiosemicarbazide ;
L1 et L2 sont choisis parmi une liaison, alkyle en C₁₋₁₀ ou polyéthylène glycol (PEG) ;
B est alcyne, azide, thiol, tétrazine, DBCO, TCO, vinyle ou méthylcyclopropène ;
C est azide, alcyne, maléimide, TCO, vinyle, méthylcyclopropène, azide ou tétrazine ;
D est un groupe choisi parmi amino et thiol ; et
E est un extincteur sombre choisi parmi Atto612Q, DABCYL, le rouge de méthyle, des colorants de diarylrhodamine QSY-7 et le perchlorate de 6-(diméthylamino)-2-[4-[4-(diméthylamino)phényl]-1,3-butadiényl]-1-éthyl-quinoléinium, un colorant de type rhodamine ou Cy5, comprenant en outre un groupe NHS-ester ou un groupe maléimide ;
et ladite molécule capturée au site de liaison d'antigène de l'anticorps est un antigène ou un fragment de celui-ci ou un peptide, qui est capable de se lier au site de liaison d'antigène de l'anticorps.

2. Conjugué selon la revendication 1, dans lequel ladite molécule capturée au site de liaison d'antigène de l'anticorps est marquée, par exemple avec un fluorophore ou une molécule de génération de signal.

3. Conjugué selon la revendication 1 ou 2, dans lequel :
lorsque B est alcyne, alors C est azide ;
lorsque B est azide, alors C est alcyne ;
lorsque B est thiol, alors C est maléimide ;
lorsque B est tétrazine, alors C est TCO, vinyle ou
méthylcyclopropène ;
lorsque B est DBCO, alors C est azide ; et
lorsque B est TCO, vinyle ou méthylcyclopropène, alors C est tétracine.

4. Conjugué selon l'une quelconque des revendications 1 à 3, dans lequel L1 et L2 sont tous deux PEG ; ou L1 est alkyle en C₁₋₁₀ et L2 est PEG ; ou L1 est PEG et L2 est alkyle en C₁₋₁₀.

5. Conjugué selon l'une quelconque des revendications 1 à 3, dans lequel les groupes chimiques A et B forment conjointement un groupe hydrazide-alcyne, dans lequel ledit groupe hydrazide-alcyne est un composé de formule (II) :
H₂N-NH-(C=O)-L1-C≡CH (II),
dans lequel L1 est une liaison ou alkyle en C₁₋₁₀.

6. Conjugué selon l'une quelconque des revendications précédentes, comprenant, essentiellement constitué de ou constitué de
• un anticorps ou un fragment de celui-ci,
• un lieur de formule (I) selon la revendication 1 ;
o dans lequel ledit lieur de formule (I) est lié à l'anticorps à l'extrémité proximale par l'intermédiaire d'un composé choisi dans
i. le groupe constitué d'hydrazide-alcyne-azide, hydrazide-azide-alcyne, hydrazide-thiol-maléimide, hydrazide-DBCO-azide, hydrazide-TCO- tétrazine, hydrazide-méthylcyclopropène-tétrazine et hydrazine-vinyl-tétrazine, dans lequel le groupe hydrazide du lieur est lié de façon covalente à une région Fc de l'anticorps ;
ii. le groupe constitué d'aminooxy-alcyne-azide, aminooxy-azide-alcyne, aminooxy-thiol-maléimide, aminooxy-DBCO-azide, aminooxy-TCO-tétrazine, aminooxy-méthylcyclopropène-tétrazine et aminooxy-vinyl-tétrazine, dans lequel le groupe aminooxy du lieur est lié de façon covalente à une région Fc de l'anticorps ; et
iii. le groupe constitué de thiosemicarbazide-alcyne-azide, thiosemicarbazide-azide-alcyne, thiosemicarbazide-thiol-maléimide, thiosemicarbazide-DBCO-azide, thiosemicarbazide-TCO-tétrazine, thiosemicarbazide-méthylcyclopropène-tétrazine et thiosemicarbazide-vinyl-tétrazine, dans lequel le groupe thiosemicarbazide du lieur est lié de façon covalente à une région Fc de l'anticorps ;
∘ dans lequel lesdits composés des groupes i. à iii. contiennent un groupe L1 selon la formule (I) telle que définie dans la revendication 1 ; et
∘ dans lequel ledit lieur comprend à l'extrémité distale un extincteur sombre choisi parmi Atto612Q, DABCYL, le rouge de méthyle, des colorants de diarylrhodamine QSY-7 et le perchlorate de 6-(diméthylamino)-2-[4-[4-(diméthylamino)phényl]-1,3-butadiényl]-1-éthyl-quinoléinium, un colorant de type rhodamine ou Cy5 ;
• une molécule capturée au site de liaison d'antigène de l'anticorps, qui comprend un fluorophore.

7. Conjugué selon l'une quelconque des revendications précédentes, comprenant, essentiellement constitué de ou constitué de
• un anticorps monoclonal,
• un lieur de formule (I) ;
∘ dans lequel ledit lieur est, à l'extrémité proximale, lié à là région Fc de l'anticorps par l'intermédiaire d'un composé choisi parmi :
▪ hydrazide-alcyne-azide ;
▪ hydrazide-tétrazine-TCO ; et
▪ aminooxy-azide-alcyne ;
∘ dans lequel L1 est PEG ou alkyle en C₁₋₁₀ et L2 est PEG
∘ et dans lequel ledit lieur comprend à l'extrémité distale l'extincteur Atto612Q ;
• une molécule capturée au site de liaison d'antigène de l'anticorps, qui comprend le fluorophore EuLH.

8. Conjugué selon l'une quelconque des revendications précédentes, comprenant, essentiellement constitué de ou constitué de
• un anticorps monoclonal,
• un lieur de formule (I) ;
∘ dans lequel ledit lieur est, à l'extrémité proximale, lié à la région Fc de l'anticorps par l'intermédiaire hydrazide d'acide 4-pentynoïque ; L2 est un PEG linéaire comprenant 5 à 7, de préférence 6 monomères d'oxyde d'éthylène ; et
∘ dans lequel ledit lieur comprend, à l'extrémité distale, l'extincteur sombre Atto612Q ;
• une molécule capturée au site de liaison d'antigène de l'anticorps, qui comprend le fluorophore EuLH.

9. Conjugué selon l'une quelconque des revendications précédentes, dans lequel l'extinction avec un analyte, un antigène ou un biomarqueur est effectuée de façon intramoléculaire.

10. Procédé de préparation du conjugué selon l'une quelconque des revendications 2 à 9, comprenant les étapes de :
i. génération de groupes aldéhyde ou cétone spécifiquement réactifs à un site au niveau du résidu asparagine à la position 297 ou une position étroitement proche de la position 297 de la région Fc de l'anticorps par oxydation de l'anticorps ;
ii. réaction de l'anticorps oxydé avec un composé A-Ll-B, de préférence choisi parmi hydrazide-Ll-alcyne, hydrazide-Ll-azide, hydrazide-Ll-thiol, hydrazide-Ll-tétrazine, hydrazide-L1-DBCO, hydrazide-Ll-TCO, hydrazide-Ll-vinyle, hydrazide-Ll-méthylcyclopropène, aminooxy-Ll-alcyne, aminooxy-Ll-azide, aminooxy-Ll-thiol, aminooxy-Ll-tétrazine, aminooxy-Ll-DBCO, aminooxy-Ll-TCO, aminooxy-Ll-vinyle, aminooxy-Ll-méthylcyclopropène, thiosemicarbazide-Ll-alcyne, thiosemicarbazide-Ll-azide, thiosemicarbazide-Ll-thiol, thiosemicarbazide-Ll-tétrazine, thiosemicarbazide-Ll-DBCO, thiosemicarbazide-Ll-TCO, thiosemicarbazide-Ll-vinyle, thiosemicarbazide-Ll-méthylcyclopropène ; dans lequel A, B et L1 sont tels que définis dans la revendication 1 ;
iii. préparation d'un composé C-L2-D-E ; dans lequel C, L2, D et E sont tels que définis dans la revendication 1 ;
iv. réaction du groupe chimique B de l'anticorps de l'étape ii., qui comprend le composé A-Ll-B, avec le composé C-L2-D-E de l'étape iii. par des réactions de chimie click ; et
v. capture d'une molécule qui est capable de se lier au site de liaison d'antigène de l'anticorps et qui comprend une molécule de génération de signal, telle qu'un fluorophore.

11. Procédé *in vitro* pour le diagnostic d'une maladie ou d'un état pathologique comprenant les étapes de :
i. mise en contact d'un conjugué selon l'une quelconque des revendications 2 à 9 avec un échantillon obtenu à partir d'un sujet suspecté d'être atteint d'une maladie ou d'une affection devant être diagnostiquée,
ii. détection de la quantité d'un analyte, antigène, biomarqueur ou similaire, ou d'une isoforme de celui-ci, dans ledit échantillon obtenu à partir dudit sujet ;
iii. comparaison de la quantité détectée dudit analyte, antigène ou biomarqueur dans ledit échantillon à une quantité de l'analyte, antigène ou biomarqueur caractéristique d'un témoin normal ;
de sorte qu'une quantité modifiée, de préférence une quantité élevée dudit analyte, antigène ou biomarqueur dans ledit échantillon par rapport au témoin normal est un indicateur positif de la maladie ou affection devant être diagnostiquée.

12. Kit de diagnostic, comprenant
• un composé A-Ll-B, de préférence choisi parmi hydrazide-L1-alcyne, hydrazide-Ll-azide, hydrazide-Ll-thiol, hydrazide-L1-tétrazine, hydrazide-Ll-DBCO, hydrazide-Ll-TCO, hydrazide-L1-vinyle, hydrazide-L1-méthylcyclopropène, aminooxy-Ll-alcyne, aminooxy-Ll-azide, aminooxy-Ll-thiol, aminooxy-Ll-tétrazine, aminooxy-Ll-DBCO, aminooxy-Ll-TCO, aminooxy-Ll-vinyle, aminooxy-Ll-méthylcyclopropène, thiosemicarbazide-Ll-alcyne, thiosemicarbazide-Ll-azide, thiosemicarbazide-Ll-thiol, thiosemicarbazide-Ll-tétrazine, thiosemicarbazide-Ll-DBCO, thiosemicarbazide-Ll-TCO, thiosemicarbazide-Ll-vinyle, thiosemicarbazide-L1-méthylcyclopropène ; dans lequel A, B et L1 sont tels que définis dans la revendication 1 ;
• un composé C-L2-D-E ; dans lequel C, L2, D et E sont tels que définis dans la revendication 2 ;
• un oxydant pour oxyder un anticorps,
• un fluorophore, et
• des instructions pour utiliser les composants dudit kit pour préparer et utiliser le conjugué selon l'une quelconque des revendications 2 à 9.

13. Kit de diagnostic comprenant un conjugué déjà assemblé selon l'une quelconque des revendications 2 à 9, une plaque de puits, telle qu'une plaque de 96 ou 384 puits, ou une puce microfluidique, dans lequel chaque puits de la plaque de puits ou la puce microfluidique est chargé avec le conjugué de l'invention, et ledit kit comprenant des échantillons témoins négatifs et positifs.

14. Conjugué selon l'une quelconque des revendications 2 à 9 pour utilisation dans le diagnostic et/ou la surveillance de l'état de maladies ou affections telles que des troubles cardiaques, une inflammation, une parathyroïdectomie, la coagulation sanguine, le syndrome métabolique et des maladies rénales.
